# EUROPEAN PATENT SPECIFICATION

(11) **EP 1 088 315 B1**
(45) Date of publication and mention of the grant of the patent: **25.06.2003**
(21) Application number: 99925247.1
(22) Date of filing: 24.05.1999
(51) Int. Cl.: H01F 1/00, B03C 1/01, G01N 33/543, A61K 49/00

(54) **NUCLEATION AND GROWTH OF MAGNETIC METAL OXIDE NANOPARTICLES AND ITS USE**
KEIMBILDUNG UND WACHSTUM VON METALLOXID-NANOPARTIKELN UND VERWENDUNG
NUCLEATION ET CROISSANCE DE NANOPARTICULES D'OXYDE METALLIQUE MAGNETIQUE ET UTILISATION DE CES DERNIERES

(30) Priority: 26.05.1998 US 84726
(43) Date of publication of application: 04.04.2001
(73) Proprietor: BAR-ILAN UNIVERSITY, IL-52900 Ramat Gan (IL)
(72) Inventor: MARGEL, Shlomo, 76210 Rehovot (IL); GURA, Sigalit, 72292 Ramla (IL)
(74) Representative: Long, Giorgio
(86) International application number: IL9900275
(87) International publication number: WO99062079

(56) References cited:
- EP-A- 0 180 384
- EP-A- 0 600 529
- US-A- 5 062 991
- AUTENSHLYUS A I ET AL: "MAGNETIC-SENSITIVE DEXTRAN-FERRITE IMMUNOSORBENTS (FOR DIAGNOSTIC AND THERAPY)" JOURNAL OF MAGNETISM AND MAGNETIC MATERIALS, vol. 122, no. 1 / 03, 1 April 1993 (1993-04-01), pages 360-363, XP000362577 ISSN: 0304-8853

## Description

### FIELD OF THE INVENTION

The present invention relates to a method for the preparation of magnetic nanoparticles by producing nuclei made of polymers which are metal chelating agents, and growing magnetic metal oxide coating thereon, to hollow magnetic nanoparticles obtained therefrom and to various uses thereof.

### BACKGROUND OF THE INVENTION

Magnetic nanoparticles have a wide range of potential applications, due to their relatively high surface area and magnetic properties. Typical examples of such applications are magnetic storage, magnetic sealing, X-ray enhancement, the manufacture of transformers, inductors, audio and video heads and a variety of biomedical applications (contrast reagents for MRI, cell labeling, cell separation, diagnostics, drug delivery, hyperthermia, oligonucleotide and peptide synthesis, detoxification of undesired molecules, immobilized enzymes, chromatography, etc).

In recent years, extensive efforts have been carried out to synthesize nanoparticles having magnetic properties. The preparation of these particles is quite complex, because it is important to obtain a substantially homogeneous population of particles of narrow size distribution, and to ensure conditions reducing to the minimal extent agglomeration processes in the solution, to name just two of the factors involved in the production of magnetic nanoparticles. In general, the methods disclosed by the art are divided into three principal categories.

The first method is based on the microencapsulation of the magnetic material within an appropriate matrix. For example, Margel et al., J. Immunol. Methods 28, 341 (1979) describe the heterogeneous polymerization of appropriate monomers in the presence of ferrofluid particles and appropriate surfactants. This procedure usually results in the formation of magnetic particles composed of ferrofluidic particles embedded in the polymer nanoparticles and coated with the surfactant to prevent agglomeration.

The second method is based on the direct precipitation of the magnetic oxides, e.g. magnetite (Fe₃O₄) and/or maghemite (γ Fe₂O₃), by contacting ferrous and ferric salts, followed by various surface treatments, as disclosed, for example, in WO 88/06632. These procedures typically result in low yields of stable particles of narrow size distribution.

The third method is based on the formation of a magnetic coating, i.e., it involves the formation of iron oxides or ferrite coating on the surface of preformed particles. M. Abe, Proceedings of The Sixth International Conference on Ferrites (ICF-6), Tokyo and Kyoto, Japan 1992, p. 472-477, S. Nagahata et al, Proceedings of The Sixth International Conference on Ferrites (ICF-6), Tokyo and Kyoto, Japan 1992, p 279-282 and M. Abe and T. Itoh, Thin Solid Films 216, 155 (1992), disclose processes for preparing magnetic nanoparticles of sizes ranging from approximately 0.3µm up to a few microns, via magnetic thin coatings of Fe₃O₄ or (Fe,M)₃O₄ [M = Fe, Co, Ni, etc.] onto preformed polyacrylate nanoparticles containing hydroxyl surface groups, i.e., nanoparticles the sizes of which are about 0.3 µm or higher, are separately prepared and then subjected to a coating process.

Another method known in the art based on the formation of a magnetic coating on a preformed particle was disclosed by Margel et al., in WO 96/11054. According to this publication, monodispersed magnetic nanoparticles of sizes ranging from ca. 0.3 µm up to a few microns are prepared via magnetic thin coatings of ferrite or iron oxides on preformed monodispersed micron-sized polystyrene particles (or similar hydrophobic particles, e.g. polychloromethylstyrene). According to this process, magnetic nanoparticles are formed by polymerization of ferrous and ferric salts on preformed micron-sized polystyrene particles coated by hydrophilic surfactants such as polyvinylpyrrolidone. This process, which produces iron oxide islands coating on the surfactant-polystyrene particles, is limited to polystyrene particles coated with hydrophilic surfactants and of sizes ranging from ca. 0.3 µm up to a few microns.

US 5,062,991 discloses a method for the preparation of ferrite nanoparticles in the presence of gelatin (type B) aqueous solution, metal salt/s such as FeCl₂, NaNO₃ as an oxidizing reagent and sodium hydroxide as an alkaline agent. According to US 5,062,991, gelatin is used for both nucleation and protection of the formed particles against agglomeration, i.e., gelatin is intended to form part of the coating. Because of this dual function of gelatin, as a support vehicle for nucleation and as stabilizer surfactant, a relatively high concentration thereof is applied. Furthermore, US 5,062,991 is specifically for gelatin type B (60 bloom) or alkali-cured. According to US 5,062,991, no other type of gelatin, e.g., type A, or polymer, e.g., dextran and polyvinylpyrrolidone, has been successfully used for preparing the magnetic particles. The process according to US 5,062,991 is alleged to yield particles of sizes ranging from 0.1µm up to 1µm, but this patent fails to exemplify the preparation of particles of diameters less than 0.2µm in satisfactory yield and in satisfactory size distribution.

It is therefore clear that the art has failed to provide a completely satisfactory method for the production of magnetic nanoparticles of narrow size distribution, in particular magnetic nanoparticles having a size lower than 0.3µm and in particular lower than 0.1µm, in good yields. Also, the methods according to the present state of the art do not enable the utilization of a variety of substrate materials on which the magnetic coating may be formed, and furthermore, the concentration of the substrate materials applied according to the art is relatively high. These critical features, namely, the size distributions obtainable according to the present state of the art and the materials and range of concentration thereof which can be employed for preparing the particles to be coated, limit significantly the scope of applications of the magnetic particles. The importance of homogeneous size distribution of the produced magnetic nanoparticles is derived from the fact that heterogeneous colloidal particles are difficult to define and characterize. Heterogeneous colloidal particles may also obscure the detection of small atypical populations, being present either at the time of manufacture or developing with storage. Atypical subpopulations of colloidal particles are considered to be potential sources of toxicity for injection into humans. Therefore, the ability to assure the absence of such subpopulations improves the quality of colloid that can be produced [see S. Palmacci, L. Josephson and E. Groman, WO 95/05669 (1995)]. The ability to obtain magnetic nanoparticles the size of which is lower than 0.1µm is also crucial, since there are several applications in which these particles are specifically suitable. For example, for MRI applications, magnetic particles of sizes below approximately 0.1µm are preferred [see S. Palmacci et al. WO 95/05669]. Another example demonstrating the advantage of magnetic particles smaller then approximately 0.1µm is in the field of specific cell separation [see S. Miltenyi et al, Cytometry 11, 231 (1990); S. Miltenyi, WO 90/07380 (1990)]. As to the use of relatively high concentrations of substrate materials on which the growth of the magnetic metal oxide is to be effected, because according to the present state of the art these materials are also intended to function as surfactant stabilizers for the nanoparticles, one skilled in the art will appreciate that this may result in various difficulties while attempting to wash-out the excess of polymeric substrates present after the formation of the final magnetic nanoparticles.

It is therefore an object of the present invention to overcome all the above-mentioned drawbacks. In particular, it is an object of the present invention to provide a method for preparing magnetic nanoparticles of sizes less than 0.3µm, in excellent yield, the size distribution of which being essentially uniform.

It is another object of the present invention to provide a method which is economically advantageous and technically superior, since it allows the application of a broad variety of polymeric substrates for the formation of the nuclei on which the magnetic coating is to be grown, the concentrations of the substrates applied may be relatively low, thereby eliminating difficulties arising from the presence of excess of polymeric materials in the solution.

### SUMMARY OF THE INVENTION

It has now been found by the inventors that magnetic nanoparticles can be produced directly by creating appropriate nuclei for growing the magnetic metal oxide thereon, and subsequently, effecting said growth of the magnetic metal oxide in a simple manner.

According to the present invention, the method for preparing the nanoparticles coated with magnetic metal oxide comprises the following steps:
a) Contacting an aqueous solution containing a soluble polymeric metal chelating agent with one or more soluble metal salts providing metal ions, wherein at least one of said metal ions is capable of forming an oxide which is magnetic, said metal ions being in amounts which do not exceed substantially the binding capacity of said chelating agent;
b) Causing said metal ions to be present in the oxidation states required for the formation of the oxide which is magnetic;
c) Maintaining the pH of the solution at the range of at least 7;
d) Introducing into the solution additional amounts of said metal salts;
e) Causing said additional metal ions to be present in the oxidation states required for the formation of the oxide which is magnetic;
f) Maintaining the pH of the solution at the range of at least 7;
g) Successively repeating the operations of step d) to f) as many times as required to obtain monodispersed nanoparticles coated with magnetic metal oxide.

By the term "nanoparticle" is meant a particle the size of which is less than 0.3 µm.

By the term "polymeric metal chelating agent" chelation of metal ions is intended. The polymeric metal chelating agents used for the formation of the nuclei on which the growth of magnetic metal oxide is to be effected, are polymers having functional groups capable of binding metal ions, such as amino, hydroxyl, carboxylate, -SH, ether, immine, phosphate and sulfide groups.

The preferred magnetic metal oxides according to the present invention are selected from the group consisting of iron oxides (particularly magnetite, maghemite or a mixture thereof) and ferrite. In order to form said iron oxides or ferrite, it is preferred that iron will be present in the solution containing the soluble metal chelating agent in two different oxidation states, Fe⁺² and Fe⁺³. Both oxidation states are caused to be present in the solution either by introducing a mixture of ferrous and ferric salts according to steps a) and d), or by introducing ferrous salts or ferric salts, and, if required, converting a portion of the Fe⁺² or Fe⁺³ to the other oxidation state, preferably by oxidation or optionally reduction, respectively. The molar ratio between Fe⁺² to Fe⁺³ ions provided in either way is preferably caused to be not higher than the ratio between Fe⁺² to Fe⁺³ ions in the composition of the desired magnetic oxide. According to a preferred embodiment of the present invention, in steps a) and d), ferrous salts are introduced into the solution, and the oxidation of a portion thereof to obtain Fe⁺³ ions is performed by introducing an oxidizer into the solution.

According to another embodiment of the present invention, other metal salts capable of forming, together with the iron ions, magnetic metal oxides, are provided in steps a) and d). These optional metals are typically selected from among transition metals.

The interaction between the various metal ions to form the magnetic metal oxide requires that the pH of the solution be at least 7. The pH is maintained within the desired range either by using an appropriate buffer solution as the aqueous solution according to step a), or by introducing a base into the solution in steps c) and f). It is preferred that once a specific pH value within said range of 7 or above is selected, it is maintained throughout the entire preparation of the magnetic nanoparticles, to ensure substantially uniform size distribution of the final products.

It should be understood that the introduction of the various components into the solution according to steps d) to g) may be carried out in two different modes of operations:
A mode of operation by increments, hereinafter referred to as a portionwise mode of operation, in which each component (metal salts, oxidizer and base) is provided in several, preferably equal, doses, which are added successively to the solution according to the order defined in steps d) to g), which steps are repeated as many times as required until the desired nanoparticles coated with the magnetic metal oxide are obtained, the quantity of each dose being such that polymerization of the metal ions in the solution, i.e., not on the particles' surface, is substantially avoided; and
a continuous mode of operation, in which each component (metal salts, oxidizer and base) is continuously added to the solution according to the order defined in steps d) to g), at an essentially uniform flow rate characteristic to each component, to avoid polymerization of the metal ions outside of the particles' surface.

The quantity of each dose according to the first mode of operation, and the flow rate according to the second mode of operation, are determined and adjusted by the experiment, as the undesired polymerization of the metal ions in the solutions, which signifies that steps d) to g) must be carried out applying smaller doses or lower flow rates, is sometimes visible to the eye, or can be identified by using conventional methods. For example, an optical method based on a coulter counter, which provides an indication that the solution has became polydispersed, may be applied. Alternatively, analytical methods such as atomic absorption may be used, to determine the build-up of significant concentration of free metal ions in the solution.

In such operating modes, the various components are assumed to be fed into the solution at a rate lower than, or similar to, the rate in which the magnetic metal oxide is formed on the surface of the nuclei, thereby avoiding the build-up of significant concentrations of said components in the solution, which may result in undesired side reactions (polymerization of metal salts outside the boundaries of the growing particles and agglomeration processes). However, regardless of the exact explanation, the fact is that operating in the above described modes yields stable, monodispersed nanoparticles coated with magnetic metal oxide of narrow size distribution.

The number of repetitions of steps d) to g) varies according to which mode of operation is selected and the desired size of the final nanoparticles, which is followed by the above described optical methods.

### BRIEF DESCRIPTION OF THE DRAWINGS

In the drawings:
Fig. 1. A scheme demonstrating the nucleation and growth of magnetic iron oxide film on a single nucleus.
Fig. 2. Titration curves of gelatin by Fe⁺² ions: (A) without the presence of an oxidizing reagent; (B) in the presence of NaNO₃ as an oxidizing reagent; (C) in the presence of NaNO₂ as an oxidizing reagent.
Fig. 3. Particle-size histograms of magnetic particles formed via a stepwise (A) and a single dose addition (B) of FeCl₂.4H₂O (0.21 mmol), NaNO₂ (0.04 mmol) and NaOH (pH 9.5) into 0.1% (w/v) gelatin aqueous solution.
Fig. 4. A SEM photomicrograph of the magnetic nanoparticles formed by portionwise addition of the polymerization reagents into the gelatin solution (0.1%).
Fig. 5. Particle-size histograms of magnetic particles formed via a stepwise (A) and a single dose addition (B) of FeCl₂.4H₂O (0.21 mmol), NaNO₂ (0.04 mmol) and NaOH (pH 9.5) into 0.3% (w/v) gelatin aqueous solution.
Fig. 6. A SEM photomicrograph of the magnetic nanoparticles formed by the single dose addition of the polymerization reagents FeCl₂.4H₂O (0.02 mmol), NaNO₃ (0.004 mmol) and NaOH (pH 9.5) into 0.3% (w/v) gelatin aqueous solution.
Fig 7. A SEM photomicrograph demonstrating the growth of the magnetic nanoparticles by portionwise additions of the polymerization reagents FeCl₂.4H₂O (0.02 mmol), NaNO₃ (0.004 mmol) and NaOH (pH 9.5) into 0.3% (w/v) gelatin aqueous solution.
Fig 8. MRI imaging of a rabbit liver without magnetic nanoparticles (A) and with magnetic nanoparticles (B).

### DETAILED DESCRIPTION OF PREFERRED EMBODIMENTS

According to the present invention, the method for preparing the nanoparticles coated with magnetic metal oxide comprises the following steps:
a) Contacting an aqueous solution containing a soluble polymeric metal chelating agent with one or more soluble metal salts providing metal ions, wherein at least one of said metal ions is capable of forming an oxide which is magnetic, said metal ions being in amounts which do not exceed substantially the binding capacity of said chelating agent;
b) Causing said metal ions to be present in the oxidation states required for the formation of the oxide which is magnetic;
c) Maintaining the pH of the solution at the range of at least 7;
d) Introducing into the solution additional amounts of said metal salts;
e) Causing said additional metal ions to be present in the oxidation states required for the formation of the oxide which is magnetic;
f) Maintaining the pH of the solution at the range of at least 7;
g) Successively repeating the operations of step d) to f) as many times as required to obtain monodispersed nanoparticles coated with magnetic metal oxide.

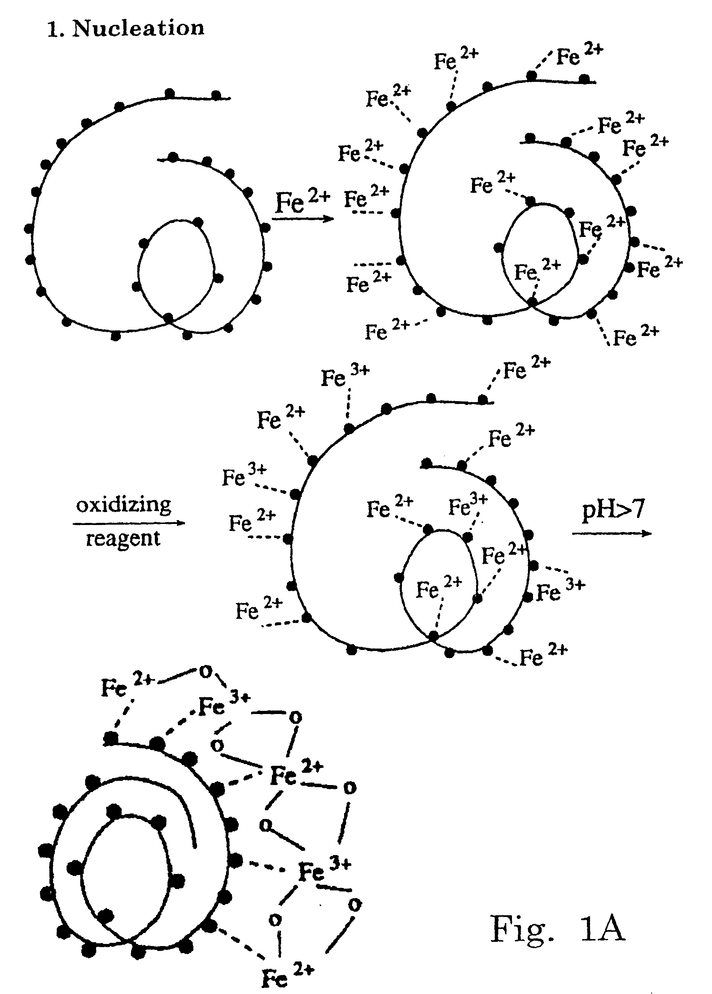

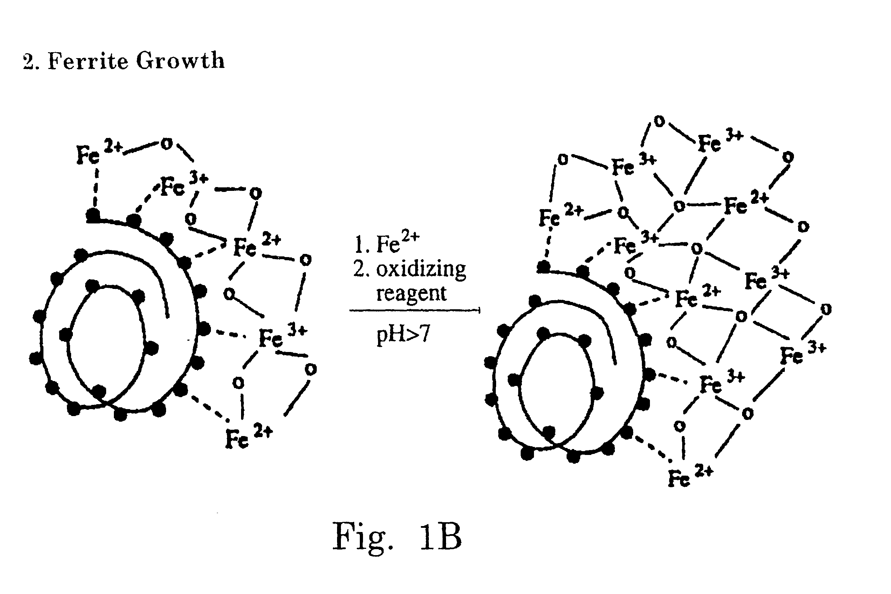

Without being bound to any theory, it is believed that via steps a) to c) nuclei are formed on which the growth of the magnetic metal oxide can be subsequently effected via steps d) to g). This mechanism is schematically given in Figure 1, wherein, for the sake of simplicity, only a single polymeric metal chelating chain is illustrated.

One aspect of the present invention therefore relates to a method for the preparation of nanoparticles coated with magnetic metal oxide, the size of which being less than 0.3µm, preferably less than 0.1µm. The magnetic nanoparticles are composed of nuclei comprising polymeric metal chelating agents on which a magnetic metal oxide is grown. One skilled in the art will appreciate that the weight % ratio between the nuclei and the magnetic coating can be controlled: the smaller the particles, the higher the percentage of the nuclei. For example, particles above about 0.2µm are composed mainly from magnetic metal oxide coating and the weight % of the nuclei is relatively negligible. On the other hand, in particles of approximately 60-70 nm, the weight % of the nuclei may range between 5-20%.

The polymeric metal chelating agents used for the formation of the nuclei on which the growth of magnetic metal oxide is to be effected, are polymers having functional groups capable of binding metal ions, the preferred groups being amino, hydroxyl, carboxylate, -SH, ether, immine, phosphate and sulfide groups. The polymeric chelating agents are most preferably selected from among the group consisting of gelatin, polymethylenimine, dextran, chitosan, polylysine and polyvinylpyrrolidone. The concentration of the polymeric metal chelating agent in the aqueous solution may vary between 0.01 and 10, preferably between 0.1 to 1 w/v.

The preferred magnetic metal oxides according to the present invention are selected from the group consisting of iron oxides (particularly magnetite, maghemite or a mixture thereof) and ferrite, (Fe,M)₃O₄, wherein M is defined hereinafter. In order to form said iron oxides or ferrite, it is preferred that iron will be present in the solution containing the soluble metal chelating agent in two different oxidation states, Fe⁺² and Fe⁺³. Both oxidation states are caused to be present in the solution either by introducing a mixture of ferrous and ferric salts according to steps a) and d), in a molar ratio which is preferably not higher than the ratio between Fe⁺² to Fe⁺³ in the composition of the desired magnetic oxide, or by introducing ferrous salts or ferric salts, and, if required, converting a portion of the Fe⁺² or Fe⁺³ to the other oxidation state, preferably by oxidation or optionally reduction, respectively, the magnitude of the portion which is oxidized or reduced being such that the resulting molar ratio between Fe⁺² to Fe⁺³ is not higher than their ratio in the composition of the desired magnetic oxide.

According to preferred embodiments of the present invention, the magnetic metal oxide coating comprises magnetite or maghemite, or a mixture thereof.

According to another preferred embodiment of the present invention, the magnetic metal oxide which constitutes the magnetic coating is ferrite. The metal salts provided in steps a) and d) may comprise, in addition to ferrous or/and ferric salts, optionally other metal salts capable of forming, together with said iron ions, the magnetic metal oxides, if ferrite coating is intended. These optional metals, designated by M, are typically selected from among transition metals, the most preferred being salts of Zn⁺², Co⁺², Mn⁺² or Ni⁺², the molar ratio Fe⁺²/M⁺ⁿ, wherein M⁺ⁿ indicates the cation of the metal M, being determined according to the stiochiometric composition of selected ferrite. The metal salts may be provided in a solid form or in the form of liquid solutions, preferred being chlorides, bromides or sulfate salts.

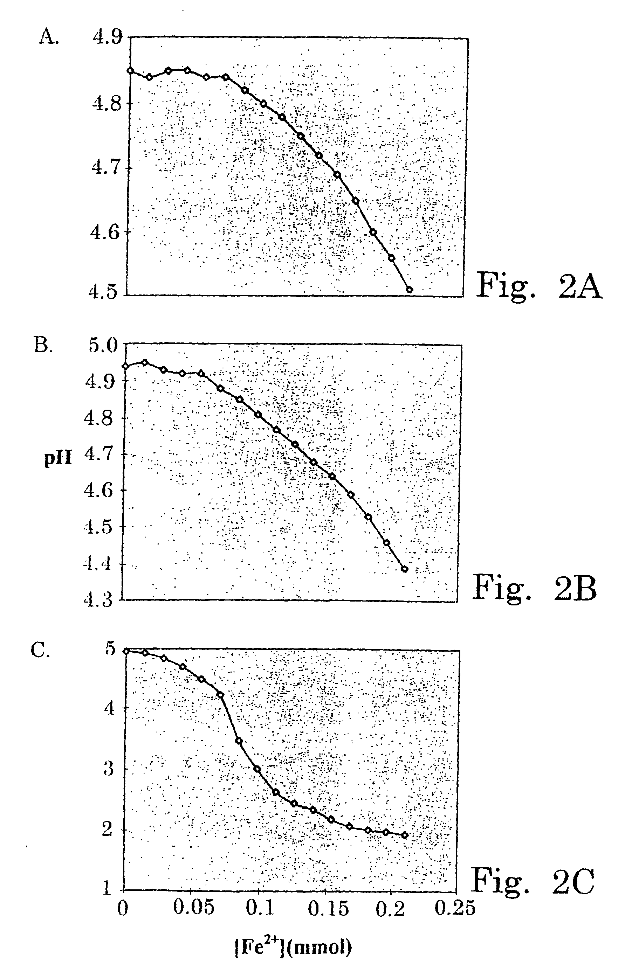

The amount of the metal ion or ions introduced into the aqueous solution according to step a) of the present invention is determined by the binding capacity of the polymeric metal chelating agent, present in the solution, with respect to said ions. By the term "binding capacity of the polymeric chelating agent" is meant the maximal amount of a certain ion which may attach to a given amount of said polymeric metal chelating agent. This parameter is of significant importance, as it has been found by the inventors that the concentration of the metal salt/s used in step a) should be similar, or not too much above the binding capacity of the chelating polymer towards said metal ions, ensuring that substantially all the nuclei are formed before a significant growth of the magnetic coating starts, in order to obtain a high yield (almost 100%) of a single population of uniform monodispersed magnetic nanoparticles. The binding capacity of a given polymeric metal chelating agent may be predetermined by methods known in the art. One such method is described in Example 1 and illustrated in Figure 2.

For producing the preferred magnetic metal oxide coating according to the present invention, it is generally preferred that the iron will be present in the solution containing the soluble metal chelating agent in two different oxidation states, Fe⁺² and Fe⁺³. In a preferred embodiment of the present invention, the metal salts provided in step a) comprise ferrous salts. In order to convert a portion of the Fe⁺² ions to Fe⁺³ according to step b), an oxidation reaction is carried out, preferably by introducing an oxidizer into the solution, whereby a portion of Fe⁺² is oxidized to Fe⁺³, said portion being such that the resulting ratio between Fe⁺² to Fe⁺³ is not higher than their ratio in the composition of the desired magnetic oxide. In a preferred embodiment of the present invention, the desired ratio between Fe⁺² to Fe⁺³ in the solution should not be higher than 1:2, and thus, not more than 2/3 of the amount of Fe⁺² introduced into said solution in steps a) or d), and preferably less, undergo the oxidation reaction. The oxidizer is preferably selected from among the group consisting of oxygen, H₂O₂, nitrite salts or nitrate salts. The preferred oxidizing reagents according to the present invention are nitrite and nitrate salts, in particular alkaline metal nitrite or nitrate salts. It has been surprisingly found by the inventors that the desired molar ratio nitrite (or nitrate) / Fe⁺² is preferably not higher than 2/3, and preferably less than 1/2, the preferred ratio being about 1/5, to ensure the desired narrow size distribution of the magnetic nanoparticles. The oxidizing reagents may be provided in a solid form or in the form of a liquid solution, optionally in the form of a basic buffer solution.

If a buffer solution is not used as the aqueous solution containing the polymeric metal chelating agent according to step a) or, optionally, as the oxidizer solution according to step b), bases should be added to the solution according to steps c) and f), to maintain in the solution a pH value of at least 7. Preferably, the pH of the solution ranges between 7 to 11, pH between 8 to 10 being most preferred. It is preferred that once a pH value within said range of 7 or above is selected, it is maintained throughout the entire preparation of the magnetic nanoparticles, to ensure substantially uniform size distribution of the final products. In another preferred embodiment of the present invention, a first pH value is maintained through step c), and a second pH value is maintained through step f), which values are within said range of at least 7, the second pH value being preferably higher. Bases which can be utilized according to the present invention may be selected from among the group consisting of alkali hydroxides, ammonia or organic bases, preferably amines.

As explained hereinbefore, steps d) to f) according to the present invention are carried out in a controlled manner, to ensure that particles of narrow size distribution are formed and side reactions (e.g. polymerization of metal salts in the aqueous solution and agglomeration processes) are avoided, by applying a mode of operations by increments or a continuous mode of operation, as explained hereinbefore. r

It should be noted that if steps d) to f) are not carried in a controlled manner as explained above, this may result in a substantial portion of agglomerated particles and/or the formation of a population of particles of broad size distribution.

The method for preparing nanoparticles coated with magnetic metal oxide according to the present invention is carried out at the temperature range below 100°C, wherein temperatures between 50°C to 90°C are generally preferred. A temperature around 60°C is typically employed.

Another aspect of the present invention relates to the preparation of hollow magnetic nanoparticles. According to this aspect, these hollow magnetic nanoparticles are obtained by removing therefrom the inner nuclei made of the polymeric metal chelating agent, by burning it off in inert atmosphere. According to a preferred embodiment, the burning off is performed by subjecting the magnetic nanoparticles to temperatures in the range 400 to 1000°C, preferably between 500 to 900°C.

Another aspect of the present invention relates to a nanoparticle the size of which is less than 0.3µm, preferably less than 0.1µm, consisting of a polymer which is metal chelating agent, coated with a magnetic metal oxide. Another aspect of the present invention relates to a hollow nanoparticle consisting of a magnetic metal oxide shell the size of which is less than 0.3µm, preferably less than 0.1µm.

Another aspect of the present invention relates to the use of the magnetic nanoparticles according to the present invention in a variety of biological and medical applications.

According to a preferred embodiment of the present invention, the biological and medical applications are selected from among the group consisting cell labeling, cell separation, controlled release, diagnostics, enzyme immobilization, protein purification, contrast agents for MRI and sono-imaging, drug delivery and chelation of heavy metal ions.

In a preferred embodiment of the present invention, molecules containing functional groups are attached to the magnetic surface of the magnetic nanoparticles of the present invention, to produce desired functional coating thereon. Preferably, said molecules comprise polymers selected from among polysaccharides, proteins, peptides, polyamines and ω-silane Si(OR)₃(CH₂)ₙX, wherein R is an alkyl substituent, n is an integer between 1 to 18, inclusive, and X is a functional group selected from among NH₂, CH₃, CN, and SH. Among the functional groups provided by said molecules, the amine group, or other functional groups which can be converted to an amine group, are of particular importance, because covalent binding of polyaldehyde ligands onto these groups may be further applied. In another preferred embodiment of the present invention, activating ligands, preferably selected from among the group consisting of acryloyl chloride, divinyl sulfone, dicarbonyl immidazole, ethylene glycolbis(sulfosuccinimidylsuccinate) and m-maleimidobenzoic acid N-hydroxysulfosuccinimide ester, which activating ligands render the surface of the nanoparticles suitable for the further coupling of bioactive compounds, are attached to the functional groups provided on the surface of the magnetic nanoparticles. Preferably, the bioactive materials are selected from among proteins, enzymes, antibodies and drugs, according to the desired biological or biomedical application.

As explained above, for some applications, e.g. specific cell separation, drug delivery, reactions in organic solvents, etc., it is essential to modify the surface of the magnetic nanoparticles in order to stabilize the particles against the agglomeration process or to introduce desired surface functional groups. The modification of the surface is accomplished by methods known in the art, which are preferably carried out in aqueous solutions. Modification of the magnetic particles with ω-alkylsilane compounds such as Si(OR)₃(CH₂)ₙX, X = CH₃, NH₂, SH, etc., were performed in aqueous solutions according to a process similar to that described by P.

Wikstrom et al., J. of Chromatography 455, 105 (1988) and by S. Brandriss et al., Langmuir 9, 1232 (1993). For example, particles bonded with ω-alkylsilane compounds containing terminal amine groups are easily suspended in polar solvents, while particles bonded with ω-alkylsilane compounds containing terminal methyl groups are easily suspended in non-polar solvents. Coating of the surface of the magnetic nanoparticles with polysaccharides such as dextran, and derivatized of dextran, e.g. dextran biotin, was usually performed by mixing the ferrite suspension in aqueous solution of the polysaccharides at high temperature (e.g. 80°C) for an appropriate period of time and then cooling it to room temperature. Cellulose-coated nanoparticles were formed by mixing at room temperature the ferrite suspension in aqueous solution of cellulose xanthate and then hydrolyzing the cellulose xanthate to cellulose by increasing the temperature of the suspension, ca. 90°C. Chitosan-coated nanoparticles were prepared by precipitating acidic chitosan aqueous solution onto ferrite coated nanoparticles kept at pH > 7, i.e. pH 9.5. The polysaccharide-coated ferrite nanoparticles were then washed from an excess of unbound polysaccharides by methods such as dialysis, gel filtration columns, magnetic field, or by high magnetic field gradient (HMFG). Hereinafter, by magnetic separation is meant using a permanent magnet or high magnetic field gradient for the separation of the nanoparticles from the solution.

According to a preferred embodiment of the present invention, coating of the surface of the magnetic nanoparticles with proteins, preferably proteins such as albumin, polylysine and a copolymer of polylysine and glutamic acid may be performed by mixing the metal oxide suspension in aqueous solution of the protein at basic pH, e.g. pH - 9.5, for an appropriate period of time. The albumin-coated magnetic nanoparticles were then washed from unbound albumin as previously described. Gelatin coated nanoparticles were prepared by mixing at high temperature (e.g. 60°C) the ferrite suspension in aqueous solution of the gelatin for an appropriate period of time. Then, the temperature was decreased to room temperature and the gelatin-coated particles were washed as previously described.

For biomedical applications such as drug delivery and controlled release, MRI and sono-imaging, the polymeric metal chelating agent and the polymers used to form the functional coating on the magnetic nanoparticle as described hereinabove, are selected from materials that are biocompatible, non-toxic and biodegradable, e.g., chelating polymers such as gelatin or chitosan.

Activated magnetic nanoparticles for the preparation of bioactive conjugated particles were prepared by interacting the functional coated particles with specific activating ligands such as carbonyl diimidazole, acryloyl chloride, divinyl sulfone [DVS], ethylene glycolbis(sulfosuccinimidylsuccinate) [sulfo-EGS] and m-maleimidobenzoic acid N-hydroxysulfosuccinimide ester [sulfo-MBS]. Residual functional groups of the functional coating of the nanoparticles were then blocked. Bioactive reagents, e.g. proteins, enzymes, antibodies, drugs, etc., were then covalently bonded to said activating ligands attached to the functional coating of the magnetic nanoparticles. Residual activating groups of the activating ligands were then blocked or hydrolized. The bioactive magnetic conjugated nanoparticles were then used for different biomedical applications. bioactive magnetic conjugated nanoparticles were then used for different biomedical applications.

Another aspect of the present invention relates to the microencapsulation of active materials within the magnetic nanoparticles, by introducing an active material, such as, for example, a drug, or a fluorescent dye, into the aqueous solution according to step a), either as such or coupled to an appropriate ligand, to produce microencapsulted magnetic nanoparticles wherein the magnetic metal oxide coating provides an enclosure for the active material.

In a further preferred embodiment, the hollow magnetic nanoparticles according to the present invention, filled with gas such as air, may be used in applications such as sono imaging as previously described for hollow nanoparticles prepared from albumin or Span 60 and Tween 80 [see M.A. Wheatley and S. Singhal, Reactive polymers 25, 157 (1995)] and ferrite coated porous silica nanoparticles [see M. Zhang, Y. Kitamoto and M. Abe, J. Phys IV France 7, C1-669 (1997).

All the above description and examples have been provided for the purpose of illustration, and are not intended to limit the invention in any way. Many modifications can be carried out in the process of the invention. For instance, different chelating polymers may be used, different ferrite compositions and different coatings may be prepared all without exceeding the scope of the invention.

### EXAMPLES.

### Example 1

### Chelating capacity of gelatin towards iron ions

The chelating capacity of gelatin (type A from porcine skin, 300 bloom - Sigma) towards iron ions was determined by titration of the gelatin in aqueous solution with iron ions in absence and in presence of oxidizing reagents such as NaNO₂ and NaNO₃. The chelating ability was measured by following the pH changes during the addition of iron ions into the gelatin solution. The pH does not change significantly as long as the iron ions chelate by gelatin. The chelating capacity of gelatin towards iron ions is indicated by a sharp decrease in the pH of the aqueous solution as a consequence of the presence of significant free iron ions in the aqueous solution.

Example 1-A. In a typical experiment, a deaerated aqueous solution (10 ml shaken at 60°C) containing 0.3% (w/v) gelatin was titrated with Fe(II) ions by stepwise addition into the gelatin solution of 10 µl portions of a reference iron solution (7 mmol FeCl₂4H₂O in 5 ml distilled water). Each added dose of the iron reference solution contained 0.014 mmol of Fe(II) ions and the time interval between each addition was ca.3 min.

Example 1-B. Experiment 1-A was repeated, except that it also included addition of the oxidizing reagent NaNO₃ (0.0028 mmol) prior to each addition of the iron solution.

Example 1-C. Experiment 1-B was repeated, substituting NaNO₃ for NaNO₂.

Figures 2:A-C indicate that under the experimental conditions, the chelating capacity of 30 mg gelatin (0.3% in 10 ml solution) towards iron ions is between 0.05 - 0.07 mmol iron ions. Under the experimental conditions, in order to form uniform magnetite nanoparticles, the addition of Fe(II) salts for the nucleation step should not significantly exceed the binding capacity of the gelatin towards the iron salts; otherwise, side reactions such as formation of polydispersed magnetite nanoparticles as well as agglutinated particles may occur.

### Example 2

### Preparation of magnetic nanoparticles with gelatin: portionwise addition versus single dose addition.

### Example 2-A: Portionwise addition

In a typical experiment, 10 ml of deaerated aqueous solution containing 0.1% (w/v) gelatin was shaken at 60°C. Solid samples containing FeCl₂.4H₂O (0.07 mmol) and NaNO₂ (0.014 mmol) were then successively added into the gelatin solution (the NaNO₂ was added immediately after the dissolution of the iron salt). The pH of the solution was then raised to 9.5 by adding NaOH solution (0.3M) into the gelatin solution. The magnetic oxide formation reaction was continued for ca. 5 min. Thereafter, the previous procedure (successive additions of FeCl₂.4H₂O, NaNO₂ and NaOH to pH 9.5) was repeated twice.

### Example 2-B: Single dose addition.

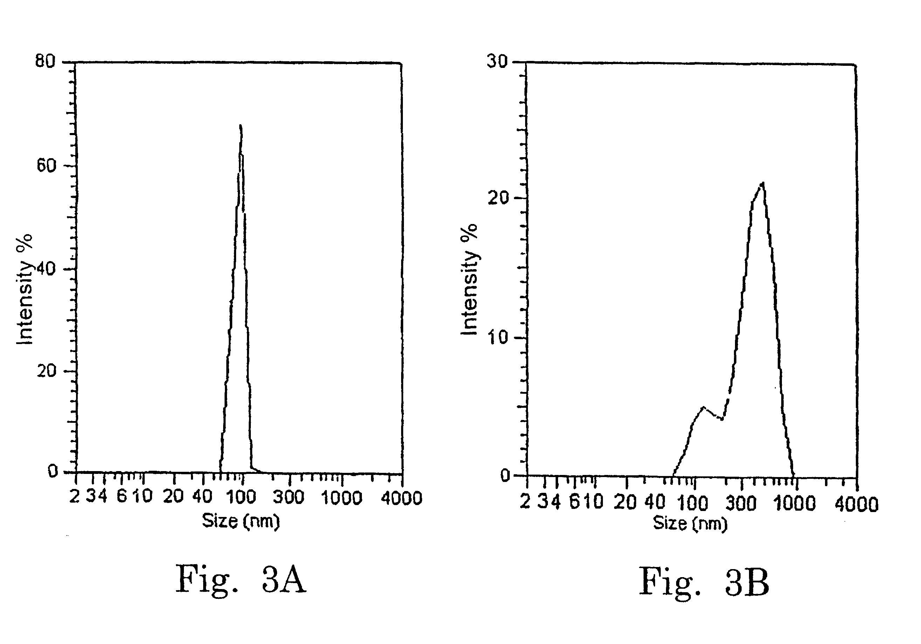

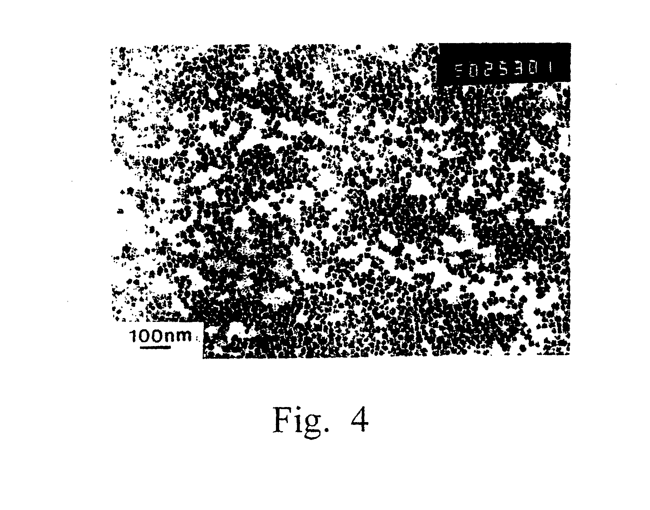

Example 2-A was repeated, substituting the three successive additions of FeCl₂.4H₂O, NaNO₂ and NaOH with a single dose addition of these reagents (0.21 mmol FeCl₂.4H₂O, 0.04 mmol NaNO₂ and then 0.3M NaOH to reach pH-9.5, reaction time -15 min, as in experiment 2-A) Table 1 and figures 3 and 4 summarize and show the major differences between experiments 2-A and 2-B.

**Table 1.**

| A comparison between the magnetic oxide formed via a single and stepwise dose additions of FeCl₂.4H₂O and NaNO₂ into the gelatin solution. | | |
|---|---|---|
| Conditions | A | B |
| [Gelatin] (% w/v) | 0.1 | 0.1 |
| [FeCl₂.4H₂O] (mmol) | 0.21 | 0.21 |
| [NaNO₂] (mmol) | 0.04 | 0.04 |
| pH | 9.5 | 9.5 |
| **No. of doses** | **3** | **1** |
| Size (by coulter, nm) | 92±15 | 477±246 |

Example 2 demonstrates the major differences of the magnetic oxide formed by the portionwise and the single dose additions of similar concentration of the polymerization reagents into the gelatin solution. The magnetic oxide nanoparticles formed by the portionwise additions were monodispersed of narrow size distribution. In the wet state, their average diameter is 92 nm as indicated by coulter measurments (Fig. 3A) and in the dry state their average diameter is ca. 15 nm as indicated by SEM measurments (Fig. 4). Almost 100% of the added iron salts were used for the magnetic coating (only an insignificant amount of the iron salts were found in the aqueous solution, apart from the particles). The magnetic nanoparticles were also very well dispersed and did not settle even during several months. On the other hand, the magnetic particles formed by the single addition were with broad size distribution and contained a high percentage of agglomerated particles. Also, the suspension was unstable and during a relatively short period of time, most of the particles precipitated, and even after severe sonication, the particles did not suspend well and very soon they agglomerated and precipitated.

### Example 3

Example 2 was repeated, substituting the oxidizing reagent NaNO₂ for NaNO₃ and the 0.1% (w/v) gelatin for 0.3% (w/v), as shown in Table 2.

**Table 2.**

| A comparison between the magnetic oxide formed via a single and stepwise dose additions of FeCl₂.4H₂O and NaNO₃ into the gelatin solution. | | |
|---|---|---|
| Conditions | A | B |
| [Gelatin] (%w/v) | 0.3 | 0.3 |
| [FeCl₂.4H₂O] (mmol) | 0.21 | 0.21 |
| [NaNO₃] (mmol) | 0.04 | 0.04 |
| pH | 9.5 | 9.5 |
| **No. of doses** | **3** | **1** |
| Size (by coulter, nm) | 65±9 | 16-800 nm (polydispersed) |

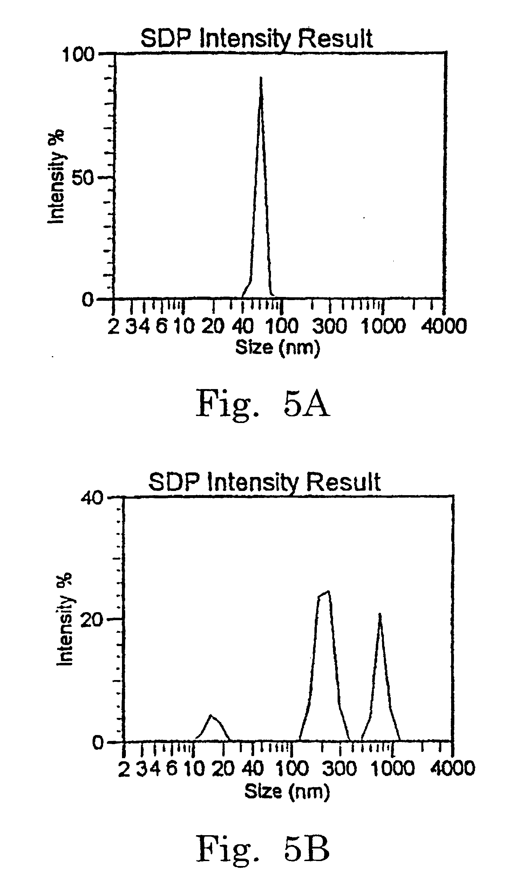

This experiment (see Table 2 and Fig. 5) demonstrated a similar behavior to that described in Example 2, i.e., a significant superiority of the magnetic nanoparticles formed by portionwise additions of the polymerization reagents into the gelatin solution, compared to a single addition of similar concentration of the polymerization reagents.

The magnetic nanoparticles prepared by the portionwise addition were monodispersed, of narrow size distribution with an average diameter of 65 nm (smaller than those formed in the presence of 0.1% gelatin (92 nm, as shown in Table 1). Almost 100% of the added iron salts were used for the magnetic coating. The magnetic nanoparticles were also very well dispersed, and did not precipitate even after several months. On the other hand, the suspension of magnetic particles formed by the single addition was unstable and contained a high percentage of agglomerated particles with very broad size distribution.

### Example 4

### Substitution of gelatin by polyethyleneimine.

Example 3 was repeated, substituting the gelatin by polyethyleneimine with an average molecular weight of 50,000. A similar difference to that described previously between the portionwise additions and the single dose addition was observed, except that the average diameter of the nanoparticles was different, e.g. 190 ± 40 nm for the particles formed by the portionwise addition and 380 ± 300 nm for the single dose addition.

### Example 5

An experiment similar to that described in example 3 was performed, substituting the solid samples- of FeCl₂.4H₂O and NaNO₃ for liquid samples. For this experiment, reference aqueous solutions containing FeCl₂.4H₂O (2.1g/5 ml 0.1N HCl) and NaNO₃ (0.5g/5mlH₂O) have been prepared and used according to the following description:
10 ml of deairated aqueous solution containing 0.3% (w/v) gelatin was shaken at 60°C. Samples containing FeCl₂.4H₂O. and NaNO₃ were then successively added into the gelatin solution (the NaNO₃ was added 1 min after the addition of the iron salt). The pH of the solution was then raised to 9.5 by adding NaOH (1.0 N). The reaction was performed portionwise (equal portions) or in a single dose, according to Table 3. The total reaction time for both the portionwise and the single dose addition was 15 min.

**Table 3.**

| A comparison between the magnetic oxide formed via single and stepwise dose additions of FeCl₂.4H₂O and NaNO₃ aqueous solutions into the gelatin solution. | | | |
|---|---|---|---|
| Conditions | A | B | C |
| [Gelatin] (% w/v) | 0.3 | 0.3 | 0.3 |
| [FeCl₂.4H₂O] (mmol) | 0.21 | 0.21 | 0.42 |
| [NaNO₃] (mmol) | 0.04 | 0.04 | 0.08 |
| pH | 9.5 | 9.5 | 9.5 |
| **No. of doses** | 3 | 1 | 1 |
| Average size (by coulter, nm) | 65 | polydispersed | polydispersed |

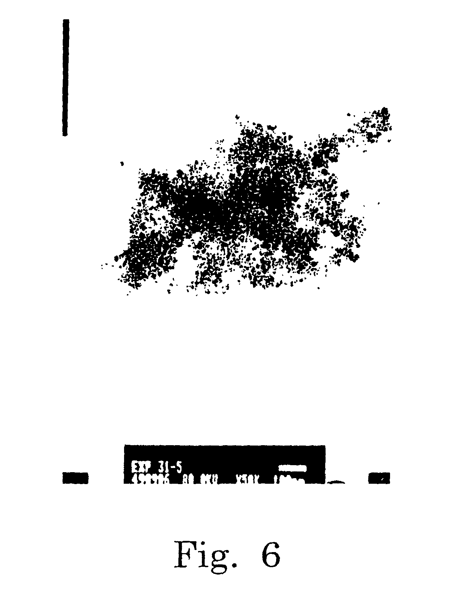

A similar difference to that described in example 3 between the portionwise additions (Table 3-A) and the single dose addition (Table 3-B & C) was observed. Fig. 6, for example, is a typical SEM picture demonstrating the agglomerated particles formed via the single dose addition according to the details of Table 3-B.

### Example 6

### Effect of pH

Example 5 was repeated, substituting pH 9.5 by pH 7.0 and by pH 11.0. Similar results were observed.

### Example 7

### Effect of temperature

Example 5 was repeated, substituting 60°C by 50°C and by 90°C. Similar results were observed.

### Example 8

### Preparation of metal ferrites

Experiment 5 was repeated substituting the Fe(II) salt for a mixture of Fe(II) salt and another metal ion selected from among Mn(II), Co(II), Ni(II) and Zn(II). The molar ratio of Fe(II)/M(II) was kept to 2/1, and most metal salts of the M(II) type were of the nitrate form. Coulter measurements demonstrated that the particles formed by the portionwise additions were of narrow size distribution, and in sizes ranging between ca. 50 nm to 150 nm, depending on the metal ion type. On the other hand, the ferrite particles formed by the single dose addition were with broad size distribution and contained a significant amount of agglomerated particles.

### Example 9

### Changing the molar ratio NO₃⁻/Fe⁺²

Example 5-A (portionwise additions) was repeated in the following molar ratios NO₃⁻/Fe⁺² 1:20; 1:5; 1:2 and 1:1. The magnetic particles formed by using the molar ratio 1:1 were with a broad size distribution and contained a significant portion of agglomerated particles. On the other hand, the magnetic nanoparticles prepared with the other molar ratios had a similar diameter (ca. 65 nm) with a narrow size distribution, and behaved similarly to the magnetic nanoparticles prepared according to Example 5-A.

### Example 10

### Preparation of fluorescent magnetic nanoparticles

Example 5-A was repeated, substituting the gelatin solution for a similar solution containing 2 mg rhodamine B isothiocyanate. Excess fluorescent reagent was then removed by dialysis. Fluorescent magnetic nanoparticles of similar properties have been formed. Fluorescent nanoparticles are also prepared by binding the desired fluorescent moleculules to the surface of the particles according to Example 23.

### Example 11

### Substituting NaOH by NH₄OH and (CH₃)₃NOH

Example 5 was repeated, substituting NaOH by NH₄OH and (CH₃)₃NOH. Similar results were obtained.

### Example 12

### Microencapsulation of a drug, e.g. adriamycin, within the magnetic nanoparticles

Example 5-A was repeated, substituting the gelatin solution by a similar solution containing 10 mg adriamycin. Excess adriamycin was then removed by a magnetic field or by dialysis. Magnetic nanoparticles containing adriamycin have been thereby formed. It is also possible to covalently bind the drug to a suitable polymer, e.g., polyethylene glycol terminated with acyl chloride group, and then repeat the previous microencapsulation procedure.

### Example 13

### Substituting the iron salt solution and the oxidant solution for a single solution containing both reagents

Example 5 was repeated, substituting the iron salt solution and the oxidant solution with a single solution containing both reagents (while keeping their concentration). Similar results were obtained.

### Example 14

### Substitution of Fe⁺² and the oxidant solution with a single solution containing a mixture of Fe⁺² and Fe⁺³ salts

Example 13 was repeated, substituing the solution containing the Fe⁺² salt and the oxidant, with a solution containing Fe⁺² and Fe⁺³ salts, their amounts being: [Fe⁺³]= [NaNO₃],[Fe⁺²]=[Fe⁺²]-[NaNO₃], the quantities on the right side being as defined in example 5. Similar results were obtained.

### Example 15

### Continuous addition of the polymerizing reagents

Example 5 was repeated, substituting the portionwise additions of the polymerizing reagents for continuous addition, according to the following conditions: 100 µl of the iron salt solution at a flow rate of 2.1 µl/min, 36 µl of the oxidizing reagent solution at a flow rate of 0.8 µl/min and 365 µl of the NaOH solution at a flow rate of 7.5 µl/min. The oxidizing reagent solution and the base solution were added ca. 30 sec. and 60 sec., respectively following the addition of the iron salt solution. The total reaction time was approximately 50 min. Similar results were obtained.

Similar results were obtained by adding the polymerizing reagents approximately three times faster, and thereby completing the coating process after approximately 16 min.

### Example 16

### Nucleation and growth of magnetic nanoparticles

In this experiment, the nucleation and growth of magnetic particles were performed similarly to that described in example 5, according to Table 4.

**Table 4.**

| Nucleation and growth of magnetic nanoparticles. | | | | |
|---|---|---|---|---|
| Conditions | A | B | C | D |
| [Gelatin] (% w/v) | 0.2 | 0.3 | 0.3 | 0.3 |
| [FeCl₂.4H₂O] (mmol) | 0.02 | 0.02 | 0.21 | 1.05 |
| [NaNO₃] (mmol) | 0.004 | 0.004 | 0.04 | 0.21 |
| pH | 9.5 | 9.5 | 9.5 | 9.5 |
| **No. of doses** | 1 | 1 | 3 | 15 |
| Average size (by coulter, nm) | 65 | 65 | 65 | 90 |

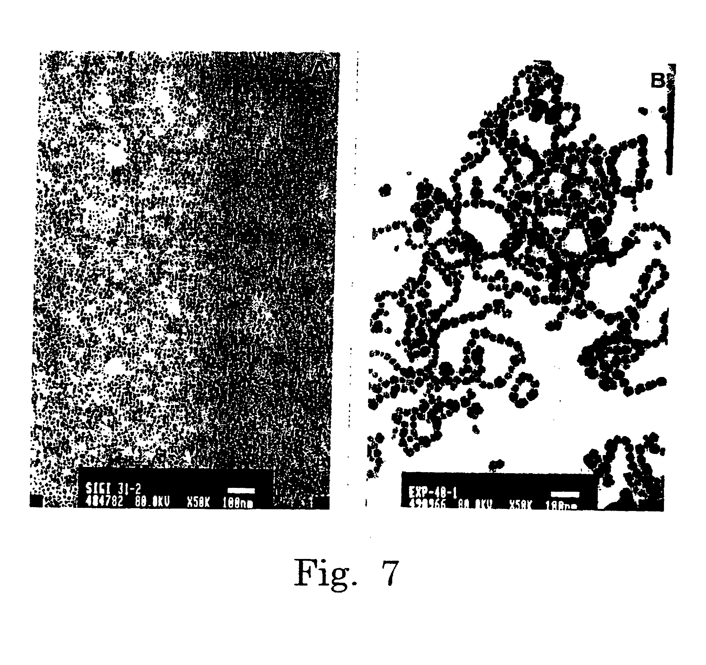

Experiments A-C demonstrate that the nucleation size is determined by the chelating polymer. The average size of the particles was 65 nm in all these trials, but the concentration of the particles of trial C was significantly higher than that of A and B. Also, in experiments A and B, the iron (II) concentration was not enough to form all the nuclei and the color of the suspension was yellow-brown due to some water soluble iron(II)-gelatin (which did not yet reach to the nucleation step). In experiment C, the concentration of the iron (II) is higher and sufficient to complete the formation of most, if not all, of the nuclei. Experiments C and D demonstrate the growth of the magnetic nanoparticles, i.e. in the wet stage from ca. 65 nm to ca. 90 nm (Table 4) and in the dry stage from ca 10 nm to ca 25 nm (Figure 7).

### Example 17

### Preparation of hollow magnetic nanoparticles

Hollow magnetic nanoparticles were prepared by burning off the organic core of the magnetic particles prepared similar to the description in example 5, according to table 5. This experiment was performed with TGA (thermogravimetric analyzer of Mettler TC11 TA processor) equipment in inert atmosphere. In each experiment 10 mg of nanoparticles were kept at 110°C for 15 min to remove water, then the temperature was raised (10°/min) to 900°C for ca. 30 min to burn off the gelatin core.

**Table 5.**

| Burning off the organic core of the magnetic nanoparticles. | | | | | |
|---|---|---|---|---|---|
| Conditions | A | B | C | D | E |
| [Gelatin] (% w/v) | 0.2 | 0.3 | 0.3 | 0.3 | 0.3 |
| [FeCl₂.4H₂O] (mmol) | 0.21 | 0.07 | 0.21 | 0.63 | 1.05 |
| [NaNO₃] (mmol) | 0.04* | 0.014 | 0.04 | 0.13 | 0.21 |
| pH | 9.5 | 9.5 | 9.5 | 9.5 | 9.5 |
| **No. of doses** | 3 | 3 | 3 | 9 | 15 |
| % burned organic core | 36 | 18 | 10 | 7 | 4 |

| | | | | | |
|---|---|---|---|---|---|
| * NaNO₂ was used instead of NaNO₃ | | | | | |

Table 5 demonstrates that the % organic core decreased as the thickness of the magnetic coating increased. Magnetic measurements have shown that the particles exhibit similar magnetic susceptibility, before and after the burning process.

### Example 18

### Preparation of magnetic nanoparticles by using PVP as the chelating polymer

Example 5 was repeated substituting gelatin with polyvinylpyrrolidone (PVP) of average m.w. of 160,000, according to table 6. Similar conclusions were observed. It was also found that it is possible to continue growing the particles of ca. 160 nm up to approximately 300nm, and that above ca. 300 nm it is difficult to prevent agglomeration processes.

**Table 6.**

| A comparison between the magnetic nanoparticles formed via a single and stepwise dose additions of FeCl₂.4H₂O and NaNO₃ aqueous solutions into PVP solution. | | | |
|---|---|---|---|
| Conditions | A | B | C |
| [PVP] (% w/v) | 1 | 1 | 1 |
| [FeCl₂.4H₂O] (mmol) | 0.07 | 0.14 | 0.14 |
| [NaNO₃] (mmol) | 0.014 | 0.028 | 0.028 |
| pH | 9.0 | 9.0 | 9.0 |
| **No. of doses** | 2 | 1 | 4 |
| Average size (by coulter, nm) | 130* | total agglomeration | 160 |

| | | | |
|---|---|---|---|
| * SEM measurements demonstred particles of narrow size distribution of average diameter of ca 5-6 nm. | | | |

### Example 19

### Preparation of magnetic nanoparticles by using dextran of different m.w. as the chelating polymers

Example 18 was repeated substituting PVP with dextran of different m.w., according to table 7.

**Table 7.**

| Effect of m.w. of dextran on the magnetic nanoparticles. | | | |
|---|---|---|---|
| Conditions | A | B | C |
| Dextran m.w. (g/mole) | 41,000 | 41,000 | 580,000 |
| [Dextran] (% w/v) | 1 | 1 | 1 |
| [FeCl₂.4H₂O] (mmol) | 0.07 | 0.21 | 0.21 |
| [NaNO₃] (mmol) | 0.014 | 0.04 | 0.04 |
| pH | 9.5 | 9.5 | 9.5 |
| **No. of doses** | 1 | 3 | 3 |
| Average size (by coulter, nm) | total agglomeration | total agglomeration | 130 |

This experiment indicates that the concentration of the added polymerizing reagents for obtaining stable monodispersed particles is highly dependent on the m.w. of the chelating polymer. For example, under similar concentrations (experiments A & B) the particles formed in the presence of dextran of the lower m.w. (41K) were unstable and contained a sustantial part of agglomerated material, while the particles formed in the presence of dextran of the higher m.w. (580K) were stable and monodispersed with relatively narrow size distribution.

### Example 20

### Magnetic measurements of the magnetic nanoparticles

Example 5 was repeated according to table 8. Magnetic susceptibility values were measured with a magnetometer (VSM Oxford Instrument Vibrating Sample Magnetometer). The magnetization curves describing the magnetic moment/ magnetic field of the particles prepared in these trials which were recorded at 250°K indicate a superparamagnetic behavior due to single domain particles.

**Table 8.**

| Magnetic susceptibility of the magnetic nanoparticles. | | | |
|---|---|---|---|
| Conditions | A | B | C |
| [Gelatin] (% w/v) | 0.3 | 0.3 | 0.3 |
| [FeCl₂.4H₂O] (mmol) | 0.07 | 0.21 | 0.63 |
| [NaNO₃] (mmol) | 0.014 | 0.04 | 0.13 |
| pH | 9.5 | 9.5 | 9.5 |
| **No. of doses** | 3 | 3 | 9 |
| Magnetic susceptibility (c.g.s.) per gram Fe | 0.036 | 0.043 | 0.025 |

### Example 21

### Lyophilization of the magnetic nanoparticles

The water washed nanoparticles containing 10% (w/v) mannitol or 1% (w/v) dextran or 0.25% (w/v) albumin prepared in examples 5-A, 8 (portionwise additions) and 17-A were lyophilized. The lyophilized powders could easily be dispersed into the original suspension by vortexing the water (or other physiological solvent) wetted powders.

### Example 22

### Surface modification of the magnetic nanoparticles

Surface modification of the magnetic nanoparticles was performed with reagents such as ω-functional alkylsilane compounds , proteins and polysaccharides. For example, the following typical surface modification procedures are hereby described:
**1.** Coating of the magnetic nanoparticles with ω-alkylsilane compounds, i.e. Si(OEt)₃(CH₂)₃NH₂.
   **1-A.** In a typical experiment, 0.1 ml of the amphiphile Si(OEt)₃(CH₂)₃NH₂ was introduced into 5 ml of the magnetic nanoparticles prepared according to examples 5-A, 8, 17 and 22 (the particles were at pH 9.5 at 60°C). The suspension was then shaken for approximately 12 h. Thereafter, the primary amine derivatized particles were washed extensively with water by a magnetic field or by dialysis. The washed coated magnetic nanoparticles were then lyophilized or stored in an aqueous solution at room temperature or at 4°C.
      Similar procedure was performed with other ω-alkylsilane compounds, e.g. Si(OEt)₃(CH₂)₃X, wherein X = CH₃, SH and epoxide. Magnetic nanoparticles coated with hydrophilic ω-groups, e.g. primary amine, dispersed easily in polar solvents (e.g.water), but produced aggragates in non-polar solvents (e.g. toluene). On the other hand, nanoparticles coated with hydrophobic ω-groups, e.g. CH₃, dispersed easily in non-polar sovents, but produced aggragates in polar solvents.
      Magnetic nanoparticles containing ω-aldehyde groups were produced by reacting the ω-primary amine derivatized particles prepared according to the previous description with 1% (w/v) glutaraldehyde at room temperature (or higher) for approximately 3 h. The ω-aldehyde derivatized particles were then washed extensively with water by a magnetic field or by dialysis and stored as previously described.
   **1-B.** In a typical experiment, 5 ml of the magnetic nanoparticles described in 1-A were dialyzed against buffer acetate, 0.1 M at pH 5.5. The suspension was then shaken for approximately 12 h at 60°C with the ω-alkylsilane compounds, i.e. Si(OEt)₃(CH₂)₃NH₂ . The washing of the derivatized particles and the derivatization with glutaraldehyde, were performed according to the previous description (1-A).
   **1-C.** In a typical experiment, 5 ml of the magnetic nanoparticles were diluted with 5 ml glycerol. 0.1 ml of the amphiphile Si(OEt)₃(CH₂)₃NH₂ was then introduced into the glycerol/water suspension. The suspension was then stirred at 60°C for approximately 3 h. Thereafter, the temperature raised to ca. 150 °C until all the water and unbound amphiphile were distilled from the glycerol suspension. The washing of the derivatized particles and the derivatization with glutaraldehyde, were performed according to the previous description (1-A).
**2.** Coating of the magnetic nanoparticles with proteins.
   Coating with BSA. In a typical experiment, 5 ml of the magnetic nanoparticles prepared according to examples 5-A, 8, 17 and 22 (pH.9.5 at 60°C) were introduced into 5 ml of 1-4% (w/v) bovine serum albumin (BSA) aqueous solution at 60°C (or at room temperature). The suspension was then shaken for 4 h at 60°C (or at room temperature) and then 12 h at room temperature. The BSA coated particles were then washed extensively with water by a magnetic field or dialysis. The washed coated magnetic nanoparticles were then lyophilized or stored in an aqueous solution at 4°C.
   Similar procedure was performed with other proteins, peptides and polyamines, e.g. human serum albumin, gelatin, casein, polylysine, copolymers of lysine and glutamic acid and polyethylenimine.
**3.** Coating of the magnetic nanoparticles with polysaccharides.
   **3-A.** Coating of the magnetic nanoparticles with dextran. In a typical experiment, 5 ml of the magnetic nanoparticles prepared according to examples 5-A, 8, 17 and 22 (pH 9.5 at 60°C) were introduced into 5 ml of 1% (w/v) dextran (MW-41K) aqueous solution at 80°C. The suspension was then shaken for 4 h at 80°C and then 12 h at room temperature. The dextran coated particles were then washed extensively with water by a magnetic field or dialysis. The washed coated magnetic nanoparticles were then lyophilized or stored in an aqueous solution at room temperature or at 4°C. A similar process was performed substituting the dextran coating at 80°C for room temperature. Similar dextran coating content was obtained.
      A similar process was performed for coating the magnetic nanoparticles with different derivatives of polysaccharides such as dextran-biotin, carboxy methylcellulose and starch.
   **3-B.** Coating of the magnetic nanoparticles with cellulose.
   **3-B-I.** In a typical experiment, 1 ml of 1% (w/v) cellulose xanthate aqueous solution was introduced into 5 ml of the magnetic nanoparticles prepared according to examples 5-A, 8 and 22 (the particles were at pH 9.5 and cooled to room temperature). The suspension was then shaken for 4 h and then heated to ca. 80°C and shaken then for another 4 h in order to hydrolyzed the xanthate groups. The cellulose coated particles were then extensively washed from free cellulose with water by a magnetic field. The washed coated magnetic nanoparticles were then lyophilized or stored in an aqueous solution at room temperature or at 4°C.
   **3-B-II.** In a typical experiment, 1 ml of 1% (w/v) cellulose xanthate aqueous solution was introduced into 5 ml of the magnetic nanoparticles prepared according to examples 5-A, 8 and 22 (the particles were at pH 9.5 and cooled to room temperature). The cellulose xanthate coated magnetic particles were then washed at room temperature with aqueous solution at pH 9.5 by a magnetic field. The suspension was then heated to ca. 80°C and treated as described in 3-B-I.
**3-C.** Coating of the magnetic nanoparticles with chitosan.
   In a typical experiment, 1 ml of 0.1-1% (w/v) chitosan aqueous solution at pH 3.0 was introduced dropwise into 5 ml of the magnetic nanoparticles prepared according to examples 5-A, 8, 17 and 22 (the particles were at pH 9.5 and cooled to room temperature). The chitosan preferebly precipitated on the nanoparticles. The chitosan coated particles were then extensively washed from free chitosan with water by a magnetic field. The washed coated magnetic nanoparticles were then lyophilized or stored in an aqueous solution at room temperature or at 4°C.
**4.** Derivatization of the magnetic nanoparticles with thiol reagents.
   Example 3-A was repeated substituting dextran for dimercaptosuccinic acid.

### Example 23

### Coupling of amino ligands (i.e. proteins and drugs) to the modified magnetic nanoparticles.

### 1. Coupling to the amine-coated nanoparticles.

In general, the amine coated magnetic nanoparticles were shaken in aqueous solution at room temperature (or other desired temperature) under optimal pH for a few hours with an appropriate activating reagent, i.e. sulfo-EGS at pH 7-8.0, acryloyl chloride at pH 8.5 and DVS at pH 9-9.5. Unbound activating reagent was then removed by a few magnetic separation cycles with the present aqueous solution and then with PBS (phosphate buffered saline 0.01M at pH 7.4), or other desired physiological solution. Residual amine groups sometimes were then blocked with acetic acid N-hydroxysuccin-imide ester by repeating the previous procedure, substituting the activating reagent with 0.2% acetic acid N-hydroxysuccin-imide ester in PBS solution [see Y. Dolitzky, S. Sturchak, B. Nizan, Ben-Ami Sela and S. Margel, Analytical Biochemistry 220, 257 (1994)]. The activated magnetic nanoparticles were then bonded with amino ligands (i.e. proteins) by shaking them at room temperature (or other desired temperature) for few hours with a desired protein and then for another few hours with a blocking reagent (e.g. glycine, ethanol amine at pH 7, etc) in PBS (or other physiological solution). Unbound protein was then removed by a few magnetic separation cycles in PBS (or other physiological solution). The conjugated nanoparticles were then kept in PBS (or water, or other physiological solution) at 4°C.

Magnetic cationic and anionic exchange resins were formed similarly substituting the proteins for ligands such as polyethylenimine, (diethylamino)ethylamine [DEAE] and amino-propionic acid.

In a typical experiment, 1 mg of the amine-coated magnetic nanoparticles of ca. 65 nm diameter in PBS (pH 7.5) were shaken at room temperature for 4 h with 0.1 mg of the activating reagent sulfo-EGS. Unbound activating reagent was then removed by magnetic separation in PBS. Residual amine groups were then blocked with acetic acid N-hydroxysuccin-imide ester by repeating the previous procedure, substituting the activating reagent with 0.2% acetic acid N-hydroxysuccin-imide ester in PBS solution The activated derivatized magnetic nanoparticles were then shaken at room temperature for 4 h with 1 mg trypsin in 5 ml PBS. Residual activating groups on the particles were then blocked by adding to the shaken conjugated nanoparticles 5 mg glycine, After another 2 h, unbound trypsin and glycine were then removed by magnetic separation in PBS. The trypsin conjugated particles were then kept in PBS (or water, or other physiological solution) at 4°C.

By using similar procedure, the following additional proteins were covalently bound to the different amine-derivatized magnetic nanoparticles: adriamycicine, goat anti-rabbit IgG (G RIgG), goat anti-mouse Ig (G MIg), goat IgG, protein A, BSA and lysozyme.

### 2. Coupling to the thiol-coated nanoparticles.

The previous procedure (1) was repeated substituting the activating groups (e.g. sulfo-EGS) for other appropriate activating reagents (e.g. sulfo-MBS) and the pH of the activation from 7-8 to 6.5-8.0.

### 3. Coupling to the aldehyde-coated nanoparticles.

In general, the aldehyde coated magnetic nanoparticles were shaken at room temperature (or other desired temperature) for few hours with the desired protein in PBS (or other physiological solution). Unbound protein was then removed by a few magnetic separation cycles in PBS. Residual aldehyde groups were then blocked with amino ligands, such as BSA, hydroxylamine or ethanol amine in physiological pH. The protein conjugated particles were then lyophilized or kept in PBS (or water, or other physiological solution) at 4°C.

Magnetic cationic and anionic exchange resins were formed similarly substituting the proteins for ligands such as polyethylenimine, (diethylamino)ethylamine [DEAE] and amino-propionic acid. For ligands containing single primary amine group, the Schiff base bonds were further reduced to single bonds with NaBH₄ or NaCNBH₄. This procedure was usually performed in ethanol suspension or in aqueous suspension at 4°C or basic pH (between 9 - 11), see A. Lazar, L.Silverstein, S.Margel and A. Mizrahi, Develop. Biol. Standard., 60, 457 (1985).

In a typical experiment, 1 mg of the aldehyde-coated magnetic nanoparticles of ca. 65 nm diameter were shaken at room temperature for 4 h with 1 mg trypsin in 5 ml PBS. Unbound trypsin was then separated by 3 magnetic separation cycles in PBS. Residual aldehyde groups on the particles were then blocked by shaking the conjugated nanoparticles at room temperature for 4 h with BSA (1%) in PBS. Unbound BSA was then removed by 3 magnetic separation cycles in PBS. The trypsin conjugated particles were then kept in PBS (or water, or other physiological solution) at 4°C.

By using similar procedure, the following additional proteins were covalently bound to the different aldehyde-derivatized magnetic nanoparticles: goat anti-rabbit IgG (G RIgG), goat anti-mouse Ig (G MIg), goat IgG, protein A, BSA and lysozyme.

### 4. Coupling to the hydroxyl-coated nanoparticles.

10 mg of the lyophilized dextran-coated nanoparticles (65 nm) were dispersed in 10 ml acetone. 1 mg carbonyl diimidazol were then added to the dispersed particles. The suspension was then shaken at room temperature for 30 min. The activated particles were then washed from unbound activating reagent by a magnetic field. The dried activated particles were then introduced at room temperature into 5 ml PBS containg 1 mg trypsin. The suspension was shaken for approximately 12 h and then rasidual activating reagent was blocked by hydrolysis at pH 8.5 (0.1 M bicarbonate solution) at room temperature for 4 h.

### Example 24

### Biological applications of the protein-conjugated magnetic nanoparticles.

### 1. Biocatalysis: proteolytic activity towards gelatin gel.

Gelatin suspension (5% w/v) in PBS was heated at 60°C for 20 min. The formed clear gelatin solution was poured into plastic dishes (5 mm diameter, 1 ml/dish) and then allowed to gel at room temperature. The weight of the gel in each dish was then accurately determined.

1 ml PBS suspension containing 20 mg trypsin-conjugated magnetic nanoparticles of approximately 65 nm diameter were applied on the gel surface. In order to demonstrate the stability of the gelatin gels towards the experimental conditions, 1 ml of PBS containing 20 mg ethanol amine-conjugated nanoparticles (control) were also added onto a similar gel surface and incubated during the experiment period of time.

Samples were incubated for ca. 48 h at 25°C. Following incubation, the gelatin gel treated with the trypsin-conjugated particles were solubilized completely. This effect is probably due to the enzymatic degradation of the gelatin by the immobilized trypsin [see S. Margel and I. Burdygin, WO 98/02189]. On the other hand, the gelatin gel incubated with the control nanoparticles remained the same. The trypsin-conjugated particles were then removed from the solubilized gelatin solution and washed with PBS by a magnetic field. This procedure was then reapplied another two times on new gelatin gels. Similar results were obtained

### 2. Controlled release.

Gelatin suspension (5% w/v) containing 5% drug such as adriamycin in PBS was heated at 60°C for 20 min. The formed clear gelatin solution was poured into plastic dishes (5 mm diameter, 1 ml/dish) and then allowed to gel at room temperature. The weight of the gel in each dish was then accurately determined. Trypsin conjugated magnetic nanoparticles were applied on the gelatin gel as described in example 24-1. During the dissolution of the gelatin adriamycin was controlled release.

### 3. Protein purification.

1 ml PBS suspension containing 10 mg goat IgG-conjugated magnetic nanoparticles of approximately 65 nm diameter were introduced into 5 ml of rabbit antisera. After approximately 10 min of shaking at room temperature, the particles were washed 3 times with PBS with a magnet. Absorbed antibodies (rabbit anti goat IgG) were then eluted from the particles with 0.2 M glycine-HCl buffer solution at pH 2.4, neutralized with NaOH and dialysed against PBS. The high purity of the eluted antibodies was then demonstrated by polyacrylamide gel electrophoresis. The magnetic conjugated nanoparticles, after treatment with glycine-HCl buffer, were washed with PBS by a magnetic field, and then stored at 4°C in the presence of sodium azide (0.05%) until reused.

### 4. Diagnostics: Determination of ₁-antitrypsin in human serum

The activity of the trypsin conjugated nanoparticles prepared as described in example 25-1 was checked with the substrate -N-benzoyl-DL-arginine p-nitroanilide (BAPNA). The conjugated trypsin in reaction with BAPNA in Tris buffer (pH-8) liberated p-nitroaniline of which its absorbance value at 400 nm was measured.

The determination of ₁-antitrypsin in human serum was based on the inhibitory effect of antitrypsin of serum on the hydrolysis of BAPNA by the conjugated trypsin in Tris buffer. The reaction is stopped by adding acetic acid, and the absorbance is then read at 400 nm. At this wavelength the liberated p-nitroaniline has a molar absorptivity of 10,500. Briefly, before the assay, each examined serum was diluted 1000 fold with Tris buffer. 2 ml of the diluted serum were then incubated at 37°C for 30 min with 1 ml suspension containing 10 mg trypsin-conjugated nanoparticles in Tris buffer. 5 ml of BAPNA solution (prepared by dissolving 100 mg BAPNA in 2.3 ml dimethylsulfoxide, and then diluted 1 ml of this stock solution with 100 ml Tris buffer at pH 8.0) at 37°C was then added for 30 min to the serum-trypsin-conjugated nanoparticles suspension. The reaction was stopped by adding 1 ml glacial acetic acid. The degree of interaction between the conjugated trypsin and BAPNA was then determined by the absorbance value at 400 nm. Control experiments were carried out with 4% human serum albumin (HSA) by using the same procedure. The precise amount of ₁-antitrypsin was determined by comparison to a standard curve obtained from a known amount of ₁-antitrypsin. The results obtained by this method were in a good agreement with the results obtained by the radial immunodiffusion method [see J. Travis and D. Johnson, Methods Enzymol. 80, 754 (1981)], the common clinical method applied for determination of ₁-antitrypsin in human serum.

### 5. (A) Cell labeling: Specific cell labeling of human red blood cells.

Fresh human red blood cells (HRBC) were shaken for 50 min at 4°C with rabbit anti-HRBC (10⁶ HRBC with 0.8 g rabbit anti-HRBC) antibodies. The sensitized cells were separated and washed 4 times by centrifugation with PBS. The washed sensitized cells were then shaken at 4°C for 1 h with G RIgG magnetic conjugated nanoparticles (5 mg , 65 nm)) prepared as described in example 24. The labeled cells were then washed with PBS from excess nanoparticles by a magnetic field. Control experiments carried out similarly, substituting the sensitized rabbit anti-HRBC with non-sensitized HRBC. Examination with SEM demonstrated the specific labeling of the RBC by the G RIgG conjugated nanoparticles. The control cells, on the other hand, were not labeled at all.

### (B) Specific labeling of mouse splenocytes bearing surface immunoglobulins (B cells).

G MIg conjugated fluorescent nanoparticles (5 mg, 65 nm) prepared as described in example 24 were shaken at 4°C for 1 h with purified mouse splenocytes (10⁶). The labeled cells were then washed from excess nanoparticles with PBS by a magnetic field. Control experiments carried out similarly, substituting the mouse splenocytes with mouse thymocytes. Examination with fluorescent microscope demonstrated the specific labeling of the mouse splenocytes with the G MIg conjugated nanoparticles. The control cells, on the other hand, were not labeled at all.

### 6. Cell separation: Separation of turkey RBC from human RBC.

A mixture containing 10⁶ human RBC and 10⁶ turkey RBC was treated with magnetic nanoparticles by using the labeling procedure described in 5-A. A magnet was then fitted on the outside wall of a vial containing 5 ml PBS suspension of the mixture of cells. Cells that were not attracted to the wall were then isolated. The attracted cells were resuspended in PBS and the magnetic separation repeated twice. Examination in light microscopy demonstrated separation efficiency of 99%-100% of the human RBC from the turkey RBC.

### 7. Chelation of heavy metal ions.

15 mg of the magnetic nanoparticles (65 nm) coated with chitosan or polyethyleneimine prepared as described in example 23 were shaken at room temperature for 1 h with 100 ml aqueous solutions containing 15 mg CdCl₂ or CuCl₂. The particles were then removed from the aqueous solution by a magnetic field. Atomic absorption measurements on the remained aqueous solution demonstrated the removal of 90-100% of the metal ions from the water.

### Example 25

### Contrast agents for MRI

Relaxation times (T1 & T2) were measured with a 2T (tesla) Whole Body MRI System (2T prestige, Elscint Ltd.) at room temperature. T1 was measured from 10 data points generated by the inversion-recovery (IR) pulse sequence. T2 was measured by the spin echo Carr-Purcell-Meiboom-Gill (CPMG) sequence. 8 echo times (TE) were used: 24, 48, 72, 104, 124, 148, 172, 204 msec (milliseconds). The repetition time (TR) was 5000 msec.

### In vitro studies:

Relaxivity measurements (R1 & R2) of magnetic nanoparticles in 5% dextrose aqueous suspension (0.05 mM Fe) prepared according to Example 5-A demonstrated values of 17.4 and 240 mM⁻¹ sec⁻¹ for R1 and R2, respectively. Similar measurements for magnetic nanoparticles prepared according to Example 5-A and coated then with dextran according to Example 23 demonstrated values of 16.2 and 202 mM⁻¹ sec⁻¹ for R1 and R2, respectively.

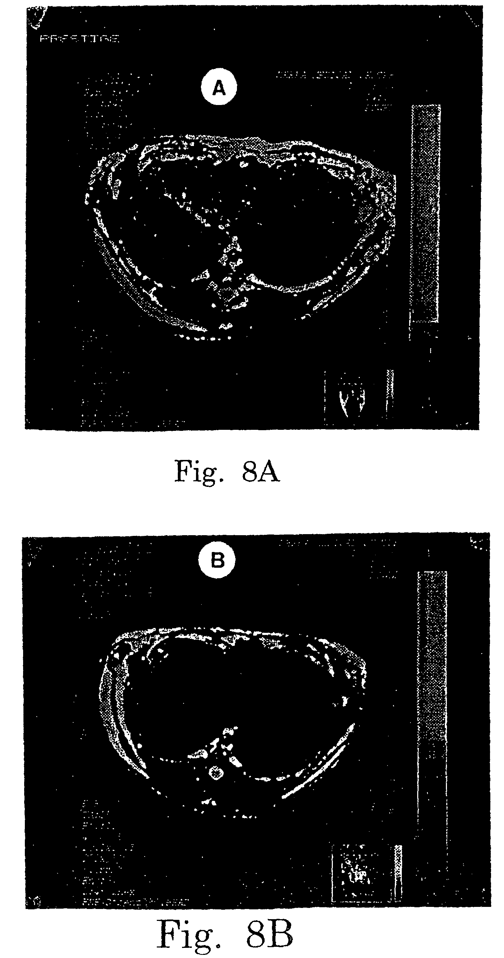

*In vivo* measurements:
MRI was performed on the liver of a rabbit at 2T using SE sequence with TR/TE = 500/96 ms. The dose injected intravenously was 10-15 µmol Fe/Kg of the formed magnetic dextrose aqueous suspension. Fig. 8A demonstrates the liver intensity before the injection of the particles and Fig. 8B demonstrates the liver intensity 25 minuets after the injection of the particles. The intensity ratio of the liver after injection (Ipost) and before injection (Ipre) of the magnetite: (Iₚₒₛₜ/Iₚᵣₑ) was found to be 0.47, indicating the high efficiency of these nanoparticles as contrast agents for MRI uses.

### Example 26

### Drug Delivery and Controlled Release

The in vivo part of example 25 was repeated, substituting the magnetic nanoparticles of example 5-A with the magnetic nanoparticles of example 12 (nanoparticles containing encapsulated drug, e.g., adriamycin). The controlled release of the drug in the liver is due to the biodegradability of the magnetic nanoparticles.

## Claims

1. A method for preparing nanoparticles coated with magnetic metal oxide, comprising the following steps:
a) Contacting an aqueous solution containing a soluble polymeric metal chelating agent with one or more soluble metal salts providing metal ions, wherein at least one of said metal ions is capable of forming an oxide which is magnetic, said metal ions being in amounts which do not exceed substantially the binding capacity of said chelating agent;
b) Causing said metal ions to be present in the oxidation states required for the formation of the oxide which is magnetic;
c) Maintaining the pH of the solution at the range of at least 7;
d) Introducing into the solution additional amounts of said metal salts;
e) Causing said additional metal ions to be present in the oxidation states required for the formation of the oxide which is magnetic;
f) Maintaining the pH of the solution at the range of at least 7;
g) Successively repeating the operations of step d) to f) as many times as required to obtain monodispersed nanoparticles coated with magnetic metal oxide.

2. A method according to claim 1, wherein the polymeric metal chelating agents have functional groups capable of binding metal ions selected from the group consisting of amino, hydroxyl, carboxylate, -SH, ether, immine, phosphate and sulfide groups.

3. A method according to claim 1, wherein the polymeric metal chelating agent is selected from the group consisting of gelatin, polymethylenimine, dextran, chitosan, polylysine and polyvinylpyrrolidone.

4. A method according to claim 3, wherein the concentration of the polymeric metal chelating agent in the aqueous solution varies between 0.01 and 10 %.w/v.

5. A method according to claim 4, wherein the concentration of the polymeric metal chelating agent in the aqueous solution varies between 0.1 to 1 % w/v.

6. A method according to claim 1, wherein the metal oxide which is magnetic is selected from the group consisting of iron oxides or ferrite.

7. A method according to claim 6, wherein the magnetic iron oxide is magnetite or maghemite, or a mixture thereof.

8. A method according to claim 1, wherein the aqueous solution is contacted with ferrous salts providing Fe⁺² ions, and Fe⁺³ ions are caused to be present in the solution by oxidizing a portion of said Fe⁺² ions.

9. A method according to claim 1, wherein the aqueous solution is contacted with a mixture of ferrous and ferric salts causing Fe⁺² and Fe⁺³ ions to be present in the solution.

10. A method according to claim 8, wherein the oxidation of a portion of Fe⁺² ions is carried out by introducing an oxidizer into the solution.

11. A method according to claims 6 and 10, wherein the magnetic metal oxide is iron oxide and the portion of Fe⁺² which is oxidized is not higher than 2/3, whereby the resulting molar ratio between Fe⁺² to Fe⁺³ in the solution is not higher than 1:2.

12. A method according to claim 11, wherein the portion of Fe⁺² which is oxidized is not higher than 1/2.

13. A method according to claim 10, wherein the oxidizer is selected from among oxygen, H₂O₂, nitrite or nitrate salts.

14. A method according to claim 13, wherein the oxidizer is NO₂⁻ or NO₃⁻.

15. A method according to claim 12, wherein the molar ratio (NO₂⁻ or NO₃⁻)/Fe⁺² is not higher than 1/2.

16. A method according to claim 6, wherein the magnetic metal oxide is ferrite, further comprising adding in steps a) and d) transition metal salts.

17. A method according to claim 1, wherein the pH is maintained in the range of at least 7 by the addition of a base.

18. A method according to claim 1, wherein the pH is maintained at a constant value in the range between 8 to 10.

19. A method according to claim 1, wherein steps d) to f) are carried out in a portionwise mode of operation as hereinbefore defined.

20. A method according to claim 1, wherein steps d) to f) are carried out in a continuous mode of operation as hereinbefore defined.

21. A method according to claim 1, wherein the size of the nanoparticles is less than 0.1µm

22. A method according to claim 1, wherein the temperature is between 50°C to 90°C.

23. A method according to claim 1, further comprising the removal of the inner polymeric metal chelating agent material to produce magnetic nanoparticles which are hollow, by burning off said polymeric material in inert atmosphere.

24. A method according to of claim 1 or 23, further comprising attaching to the magnetic surface of the magnetic nanoparticles molecules containing functional groups to produce desired functional coating on the particles.

25. A method according to claim 24 wherein the molecules containing functional groups comprise polymers selected from the group consisting of polysaccharides, proteins, peptides, polyamines and ω-silane Si(OR)₃(CH₂)ₙX, wherein R is an alkyl substituent, n is an integer between 1 to 18, inclusive, and X is a functional group selected from the group consisting of NH₂, CH₃, CN, and SH.

26. A method according to claim 25, further comprising binding polyaldehyde ligands to the amine groups of the functional coating.

27. A method according to claims 25 and 26, further comprising attaching activating ligands to the functional groups capable of binding bioactive agents.

28. A method according to claim 27 wherein the activating ligands are selected from the group consisting of acryloyl chloride, divinyl sulfone, dicarbonyl immidazole, ethylëne glycolbis(sulfosuccinimidylsuccinate) and m-maleimidobenzoic acid N-hydroxysulfosuccinimide ester.

29. A method according to claim 28, further comprising coupling bioactive agents to the activating ligands.

30. A method according to claim 19 wherein the bioactive agents are compounds selected from the group consisting of proteins, enzymes, antibodies and drugs.

31. A method according to claim 1 for the microencapsulation of active materials within the magnetic nanoparticles, **characterized in that** an active material is introduced into the aqueous solution according to step a).

32. A method according to claim 31, wherein the active material is a drug or fluorescent dye.

33. A nanoparticle the size of which is less than 0.3µm, consisting of a polymer which is metal chelating agent, coated with a magnetic metal oxide.

34. A nanoparticle according to claim 33, wherein its size is less than 0.1µm.

35. A nanoparticle according to claim 34, wherein its size is less than 92 nm.

36. A hollow nanoparticle consisting of a magnetic metal oxide shell the size of which is less than 0.3µm.

37. A hollow nanoparticle according to claim 36, wherein its size is less than 0.1µm.

38. A magnetic nanoparticle according to any of claims 34 to 37, further comprising a coating of a functional polymer on the magnetic coating.

39. A magnetic nanoparticle according to claim 38 wherein the functional polymeric coating comprises polymers selected from the group consisting of polysaccharides, proteins, peptides, polyamines and ω-silane compounds.

40. A magnetic nanoparticle according to claim 39, bonded with activating ligands.

41. A magnetic nanoparticle according to claim 40 wherein the activating ligands are provided by compounds selected from the group consisting of acryloyl chloride, divinyl sulfone, dicarbonyl immidazole, ethylene glycolbis(sulfosuccinimidylsuccinate) and m-maleimidobenzoic acid N-hydroxysulfosuccinimide ester.

42. A magnetic nanoparticle according to claim 41 coupled to bioactive agents.

43. A magnetic nanoparticle according to claim 42 wherein the bioactive agent is a compound selected from the group consisting of proteins, enzymes, antibodies and drugs.

44. Microencapsule comprising a magnetic nanoparticle according to claim 33, 34 or 35, wherein an active material is enclosed within the magnetic metal oxide coating.

45. Use of the magnetic nanoparticles according to any of claims 33 to 43 for the preparation of a magnetic or medical composition for biological or medical applications.

46. Use of a magnetic nanoparticle according to claim 45, wherein said biological and medical applications are selected from the group consisting of cell labeling, cell separation, controlled release, diagnostics, enzyme immobilization, protein purification, drug delivery, contrast agents for MRI and sono-imaging applications and chelation of heavy metal ions.

## Patentansprüche

1. Verfahren zur Herstellung von mit einem magnetischen Metalloxid überzogenen Nanopartikeln, das die folgenden Stufen umfasst:
a) Kontaktieren einer wässrigen Lösung, die einen löslichen polymeren Metallchelatbildner enthält, mit einem oder mehreren, Metallionen liefernden löslichen Metallsalzen, wobei mindestens eines der Metallionen zur Bildung eines Oxids, das magnetisch ist, fähig ist, wobei die Metallionen in Mengen vorhanden sind, die das Bindungsvermögen des Chelatbildners nicht wesentlich übersteigen;
b) Bewirken, dass die Metallionen in den für die Bildung des Oxids, das magnetisch ist, erforderlichen Oxidationsstufen vorhanden sind;
c) Halten des pH-Werts der Lösung in einem Bereich von mindestens 7;
d) Einführen von weiteren Mengen der Metallsalze in die Lösung;
e) Bewirken, dass die weiteren Metallionen in den für die Bildung des Oxids, das magnetisch ist, erforderlichen Oxidationsstufen vorhanden sind;
f) Halten des pH-Werts der Lösung in einem Bereich von mindestens 7;
g) aufeinanderfolgendes Wiederholen der Vorgänge von Stufe d) bis Stufe f) so oft dies erforderlich ist, um mit einem magnetischen Metalloxid überzogene monodisperse Nanopartikel zu erhalten.

2. Verfahren nach Anspruch 1, wobei die polymeren Metallchelatbildner funktionelle Gruppen besitzen, die Metallionen, die aus der aus Amino-, Hydroxyl-, Carboxylat-, -SH-, Ether-, Imin-, Phosphat- und Sulfidgruppen bestehenden Gruppe ausgewählt sind, binden können.

3. Verfahren nach Anspruch 1, wobei der polymere Metallchelatbildner aus der aus Chelatine, Polymethylenimin, Dextran, Chitosan, Polylysin und Polyvinylpyrrolidon bestehenden Gruppe ausgewählt ist.

4. Verfahren nach Anspruch 3, wobei die Konzentration des polymeren Metallchelatbildners in der wässrigen Lösung zwischen 0,01 und 10 % (Gew./V.) variiert.

5. Verfahren nach Anspruch 4, wobei die Konzentration des polymeren Metallchelatbildners in der wässrigen Lösung zwischen 0,1 und 1 % (Gew./V.) variiert.

6. Verfahren nach Anspruch 1, wobei das Metalloxid, das magnetisch ist, aus der aus Eisenoxiden oder Ferrit bestehenden Gruppe ausgewählt ist.

7. Verfahren nach Anspruch 6, wobei das magnetische Eisenoxid Magnetit oder Maghemit oder ein Gemisch derselben ist.

8. Verfahren nach Anspruch 1, wobei die wässrige Lösung mit Fe⁺²-Ionen liefernden Eisen(II)-Salzen kontaktiert wird, und durch Oxidieren eines Teils dieser Fe⁺²-Ionen bewirkt wird, dass Fe⁺³-Ionen in der Lösung vorhanden sind.

9. Verfahren nach Anspruch 1, wobei die wässrige Lösung mit einem Gemisch von Eisen(II)- und Eisen(III)-Salzen kontaktiert wird, was bewirkt, dass Fe⁺²- und Fe⁺³-Ionen in der Lösung vorhanden sind.

10. Verfahren nach Anspruch 8, wobei das Oxidieren eines Teils der Fe⁺²-Ionen durch Einführen eines Oxidationsmittels in die Lösung durchgeführt wird.

11. Verfahren nach den Ansprüchen 6 und 10, wobei das magnetische Metalloxid Eisenoxid ist und der Teil von Fe⁺², der oxidiert wird, nicht großer als 2/3 ist, wodurch das in der Lösung erhaltene Molverhältnis von Fe⁺² zu Fe⁺³ nicht größer als 1:2 ist.

12. Verfahren nach Anspruch 11, wobei der Teil von Fe⁺², der oxidiert wird, nicht größer als 1/2 ist.

13. Verfahren nach Anspruch 10, wobei das Oxidationsmittel aus Sauerstoff, H₂O₂, Nitrit- oder Nitratsalzen ausgewählt ist.

14. Verfahren nach Anspruch 13, wobei das Oxidationsmittel NO₂⁻ oder NO₃⁻ ist.

15. Verfahren nach Anspruch 12, wobei das Molverhältnis (NO₂⁻ oder NO₃⁻)/Fe⁺² nicht größer als 1/2 ist.

16. Verfahren nach Anspruch 6, wobei das magnetische Metalloxid Ferrit ist, wobei das Verfahren ferner die Zugabe von Übergangsmetallsalzen in den Stufen a) und d) umfasst.

17. Verfahren nach Anspruch 1, wobei der pH-Wert durch die Zugabe einer Base in einem Bereich von mindestens 7 gehalten wird.

18. Verfahren nach Anspruch 1, wobei der pH-Wert auf einem konstanten Wert im Bereich zwischen 8 und 10 gehalten wird.

19. Verfahren nach Anspruch 1, wobei die Stufen d) bis f). in einem, wie im vorhergehenden angegebenen, portionsweisen Arbeitsverfahren durchgeführt werden.

20. Verfahren nach Anspruch 1, wobei die Stufen d) bis f) in einen, wie im vorhergehenden angegebenen, kontinuierlichen Arbeitsverfahren durchgeführt werden.

21. Verfahren nach Anspruch 1, wobei die Größe der Nanopartikel weniger als 0,1 µm beträgt.

22. Verfahren nach Anspruch 1, wobei die Temperatur zwischen 50 °C und 90 °C beträgt.

23. Verfahren nach Anspruch 1, das ferner das Entfernen des inneren polymeren Metallchelatbildnermaterials unter Bildung magnetischer Nanopartikel, die hohl sind, durch Wegbrennen des polymeren Materials in einer inerten Atmosphäre umfasst.

24. Verfahren nach Anspruch 1 oder 23, das ferner das Befestigen von funktionelle Gruppen enthaltenden Molekülen an der magnetischen Oberfläche der magnetischen Nanopartikel zur Bildung eines gewünschten funktionellen Überzugs auf den Partikeln umfasst.

25. Verfahren nach Anspruch 24, wobei die funktionelle Gruppen enthaltenden Moleküle Polymere umfassen, die aus der aus Polysacchariden, Proteinen, Peptiden, Polyaminen und ω-Silan Si(OR)₃(CH₂)ₙX, worin R ein Alkylsubstituent ist, n eine ganze Zahl zwischen 1 und einschließlich 18 ist und X eine aus der aus NH₂, CH₃, CN und SH bestehenden Gruppe ausgewählte funktionelle Gruppe ist, bestehenden Gruppe ausgewählt sind.

26. Verfahren nach Anspruch 25, das ferner das Binden von Polyaldehydliganden an die Amingruppen des funktionellen Überzugs umfasst.

27. Verfahren nach den Ansprüchen 25 und 26, das ferner das Befestigen von Aktivierungsliganden, die zur Bindung von biologisch aktiven Mitteln fähig sind, an den funktionellen Gruppen umfasst.

28. Verfahren nach Anspruch 27, wobei die Aktivierungsliganden aus der aus Acryloylchlorid, Divinylsulfon, Dicarbonylimidazol, Ethylenglykolbis(sulfosuccinimidylsuccinat) und m-Maleimidobenzoesäure-N-hydroxysulfosuccinimidester bestehenden Gruppe ausgewählt sind.

29. Verfahren nach Anspruch 28, das ferner die Kopplung von biologisch wirksamen Mitteln an die Aktivierungsliganden umfasst.

30. Verfahren nach Anspruch 29, wobei die biologisch aktiven Mittel aus der aus Proteinen, Enzymen, Antikörpern und Arzneimitteln bestehenden Gruppe ausgewählte Verbindungen sind.

31. Verfahren nach Anspruch 1 zur Mikroverkapselung von aktiven Materialien in den magnetischen Nanopartikeln, **dadurch gekennzeichnet, dass** ein aktives Material in die wässrige Lösung gemäß Stufe a) eingeführt wird.

32. Verfahren nach Anspruch 31, wobei das aktive Material ein Arzneimittel oder ein Fluoreszenzfarbstoff ist.

33. Nanopartikel einer Größe von weniger als 0,3 µm, das aus einem mit einem magnetischen Metalloxid überzogenen Polymer, das ein Metallchelatbildner ist, besteht.

34. Nanopartikel nach Anspruch 33, wobei dessen Größe geringer als 0,1 µm ist.

35. Nanopartikel nach Anspruch 34, wobei dessen Größe geringer als 92 nm ist.

36. Hohles Nanopartikel, das aus einer Hülle aus einem magnetischen Metalloxid besteht, wobei die Größe des Nanopartikels geringer als 0,3 µm ist.

37. Hohles Nanopartikel nach Anspruch 36, wobei dessen Größe geringer als 0,1 µm ist.

38. Magnetisches Nanopartikel nach einem der Ansprüche 34 bis 37, das ferner auf dem magnetischen Überzug einen Überzug aus einem funktionellen Polymer umfasst.

39. Magnetisches Nanopartikel nach Anspruch 38, wobei der funktionelle Polymerüberzug Polymere umfasst, die aus der aus Polysacchariden, Proteinen, Peptiden, Polyaminen und ω-Silanverbindungen bestehenden Gruppe ausgewählt sind.

40. Magnetisches Nanopartikel nach Anspruch 39, an das Aktivierungsliganden gebunden sind.

41. Magnetisches Nanopartikel nach Anspruch 40, wobei die Aktivierungsliganden durch aus der aus Acryloylchlorid, Divinylsulfon, Dicarbonylimidazol, Ethylenglykolbis(sulfosuccinimidylsuccinat) und m-Maleimidobenzoesäure-N-hydroxysulfosuccinimidester bestehenden Gruppe ausgewählte Verbindungen bereitgestellt werden.

42. Magnetisches Nanopartikel nach Anspruch 41, das an biologisch aktive Mittel gekoppelt ist.

43. Magnetisches Nanopartikel nach Anspruch 42, wobei das biologisch aktive Mittel eine aus der aus Proteinen, Enzymen, Antikörpern und Arzneimitteln bestehenden Gruppe ausgewählte Verbindung ist.

44. Mikrokapsel, die ein magnetisches Nanopartikel nach Anspruch 33, 34 oder 35 umfasst, wobei ein aktives Material in dem Überzug aus einem magnetischen Metalloxid eingeschlossen ist.

45. Verwendung der magnetischen Nanopartikel nach einem der Ansprüche 33 bis 43 zur Herstellung einer diagnostischen oder medizinischen Zusammensetzung für biologische oder medizinische Anwendungen.

46. Verwendung eines magnetischen Nanopartikels nach Anspruch 45, wobei die biologischen und medizinischen Anwendungen ausgewählt sind aus der aus Zellmarkierung, Zelltrennung, gesteuerte Freisetzung, Diagnostika, Enzymimmobilisierung, Proteinreinigung, Arzneimittelabgabe, Kontrastmittel für MRI und Ultraschallbildgebungsanwendungen und Chelatbildung von Schwermetallionen bestehenden Gruppe.

## Revendications

1. Procédé de préparation de nanoparticules enrobées d'un oxyde métallique magnétique, comprenant les étapes suivantes consistant à :
a) mettre en contact une solution aqueuse contenant un agent chélatant de métaux polymère soluble avec un ou plusieurs sels métalliques solubles fournissant des ions métalliques, où au moins l'un desdits ions métalliques est capable de former un oxyde qui est magnétique, lesdits ions métalliques étant présents en des quantités qui ne dépassent pratiquement pas la capacité de liaison dudit agent chélatant ;
b) faire que lesdits ions métalliques soient présents dans les états d'oxydation nécessaires à la formation de l'oxyde qui est magnétique ;
c) maintenir le pH de la solution dans le domaine d'au moins 7 ;
d) introduire dans la solution des quantités supplémentaires desdits sels métalliques ;
e) faire que lesdits ions métalliques supplémentaires soient présents dans les états d'oxydation nécessaires à la formation de l'oxyde qui est magnétique ;
f) maintenir le pH de la solution dans le domaine d'au moins 7 ;
g) répéter successivement les opérations des étapes d) à f) autant de fois que cela est nécessaire pour obtenir des nanoparticules monodispersées enrobées d'un oxyde métallique magnétique.

2. Procédé selon la revendication 1, dans lequel les agents chélatants de métaux polymères contiennent des groupes fonctionnels capables de lier des ions métalliques choisis dans le groupe constitué par les groupes amino, hydroxyle, carboxylate, -SH, éther, imine, phosphate et sulfure.

3. Procédé selon la revendication 1, dans lequel l'agent chélatant de métaux polymère est choisi dans le groupe constitué par la gélatine, le polyméthylènimine, le dextrane, le chisosan, la polylysine et la polyvinylpyrrolidone.

4. Procédé selon la revendication 3, dans lequel la concentration en agent chélatant de métaux polymère de la solution aqueuse varie entre 0,01 et 10 % en poids/volume.

5. Procédé selon la revendication 4, dans lequel la concentration en agent chélatant de métaux polymère de la solution aqueuse varie entre 0,1 et 1 % en poids/volume.

6. Procédé selon la revendication 1, dans lequel l'oxyde métallique qui est magnétique est choisi dans le groupe constitué par les oxydes de fer ou le ferrite.

7. Procédé selon la revendication 6, dans lequel l'oxyde de fer magnétique est la magnétite ou la maghémite, ou un mélange de ceux-ci.

8. Procédé selon la revendication 1, dans lequel la solution aqueuse est mise en contact avec des sels ferreux fournissant des ions Fe²⁺, et des ions Fe³⁺ sont présents dans la solution par oxydation d'une partie desdits ions Fe²⁺.

9. Procédé selon la revendication 1, dans lequel la solution aqueuse est mise en contact avec un mélange de sels ferreux et ferriques, entraînant la présence d'ions Fe²⁺ et Fe³⁺ dans la solution.

10. Procédé selon la revendication 8, dans lequel l'oxydation d'une partie des ions Fe²⁺ est réalisée par introduction d'un oxydant dans la solution.

11. Procédé selon les revendications 6 et 10, dans lequel l'oxyde métallique magnétique est un oxyde de fer, et la partie des ions Fe²⁺ qui est oxydée n'est pas supérieure à 2/3, afin que le rapport molaire résultant des ions Fe²⁺ à Fe³⁺ dans la solution ne soit pas supérieur à 1:2.

12. Procédé selon la revendication 11, dans lequel la partie des ions Fe²⁺ qui est oxydée n'est pas supérieure à ½.

13. Procédé selon la revendication 10, dans lequel l'oxydant est choisi parmi l'oxygène, H₂O₂, un sel nitrite ou nitrate.

14. Procédé selon la revendication 13, dans lequel l'oxydant est NO₂⁻ ou NO₃⁻.

15. Procédé selon la revendication 12, dans lequel le rapport molaire de (NO₂⁻ ou NO₃⁻)/Fe²⁺ n'est pas supérieur à ½.

16. Procédé selon la revendication 6, dans lequel l'oxyde métallique magnétique est un ferrite, comprenant en outre l'addition dans les étapes a) et d) de sels de métaux de transition.

17. Procédé selon la revendication 1, dans lequel le pH est maintenu dans le domaine d'au moins 7 par addition d'une base.

18. Procédé selon la revendication 1, dans lequel le pH est maintenu à une valeur constante dans le domaine compris entre 8 et 10.

19. Procédé selon la revendication 1, dans lequel les étapes d) à f) sont réalisées selon un mode opératoire par étapes comme défini ci-dessus.

20. Procédé selon la revendication 1, dans lequel les étapes d) à f) sont réalisées selon un mode opératoire continu comme défini ci-dessus.

21. Procédé selon la revendication 1, dans lequel la taille des nanoparticules est inférieure à 0,1 µm.

22. Procédé selon la revendication 1, dans lequel la température est comprise entre 50°C et 90°C.

23. Procédé selon la revendication 1, comprenant en outre l'élimination du matériau d'agent chélatant de métaux polymère interne pour produire des nanoparticules magnétiques qui sont creuses, par élimination par brûlage dudit matériau polymère dans une atmosphère inerte.

24. Procédé selon la revendication 1 ou 23, comprenant en outre la fixation, à la surface magnétique des nanoparticules magnétiques, de molécules contenant des groupes fonctionnels pour produire un enrobage fonctionnel souhaité sur les particules.

25. Procédé selon la revendication 24, dans lequel les molécules contenant des groupes fonctionnels comprennent des polymères choisis dans le groupe constitué par les polysaccharides, les protéines, les peptides, les polyamides et un ω-silane Si(OR)₃(CH₂)ₙX, où R est un substituant alkyle, n est un nombre entier compris entre 1 et 18, bornes comprises, et X est un groupe fonctionnel choisi dans le groupe constitué par NH₂, CH₃, CH et SH.

26. Procédé selon la revendication 25, comprenant en outre la liaison de ligands de type polyaldéhyde aux groupes amine de l'enrobage fonctionnel.

27. Procédé selon les revendications 25 et 26, comprenant en outre la fixation de ligands d'activation sur les groupes fonctionnels capables de lier des agents bioactifs.

28. Procédé selon la revendication 27, dans lequel les ligands d'activation sont choisis dans le groupe constitué par le chlorure d'acryloyle, la divinylsulfone, le dicarbonylimidazole, l'éthylèneglycol-bis(succinate de sulfosuccinidyle) et le N-hydroxysulfosuccinimideester de l'acide m-maléimidobenzoique.

29. Procédé selon la revendication 28, comprenant en outre le couplage d'agents bioactifs aux ligands d'activation.

30. Procédé selon la revendication 19, dans lequel les agents bioactifs sont des composés choisis dans le groupe constitué par les protéines, les enzymes, les anticorps et les médicaments.

31. Procédé selon la revendication 1, pour la microencapsulation de matières actives à l'intérieur des nanoparticules magnétiques, **caractérisé en ce que** la matière active est introduite dans la solution aqueuse selon l'étape a).

32. Procédé selon la revendication 31, dans lequel le matière active est un médicament ou un colorant fluorescent.

33. Nanoparticule dont la taille est inférieure à 0,3 µm, constituée d'un polymère qui est un agent chélatant de métaux, enrobé d'un oxyde métallique magnétique.

34. Nanoparticule selon la revendication 33, dans laquelle sa taille est inférieure à 0,1 µm.

35. Nanoparticule selon la revendication 34, dans laquelle sa taille est inférieure à 92 nm.

36. Nanoparticule creuse constituée d'une enveloppe d'oxyde métallique magnétique dont la taille est inférieure à 0,3 µm.

37. Nanoparticule creuse selon la revendication 36, dans laquelle sa taille est inférieure à 0,1 µm.

38. Nanoparticule magnétique selon l'une quelconque des revendications 34 à 37, comprenant en outre un enrobage d'un polymère fonctionnel sur l'enrobage magnétique.

39. Nanoparticule magnétique selon la revendication 38, dans laquelle l'enrobage polymère fonctionnel comprend des polymères choisis dans le groupe constitué par les polysaccharides, les protéines, les peptides, les polyamides et les composés ω-silane.

40. Nanoparticule magnétique selon la revendication 39, liée à des ligands d'activation.

41. Nanoparticule magnétique selon la revendication 40, dans laquelle les ligands d'activation sont fournis par des composés choisis dans le groupe constitué par le chlorure d'acryloyle, la divinylsulfone, le dicarbonylimidazole, l'éthylèneglycol-bis(succinate de sulfosuccinidyle) et le N-hydroxysulfosuccinimideester de l'acide m-maléimidobenzoïque.

42. Nanoparticule magnétique selon la revendication 41, couplée à des agents bioactifs.

43. Nanoparticule magnétique selon la revendication 42, dans laquelle l'agent bioactif est un composé choisi dans le groupe constitué par les protéines, les enzymes, les anticorps et les médicaments.

44. Microcapsule comprenant une nanoparticule magnétique selon la revendication 33, 34 ou 35, dans laquelle une matière active est incorporée à l'intérieur de l'enrobage d'oxyde métallique magnétique.

45. Utilisation des nanoparticules magnétiques selon l'une quelconque des revendications 33 à 43, pour la préparation d'une composition diagnostique ou médicale pour des applications biologiques ou médicales.

46. Utilisation d'une nanoparticule magnétique selon la revendication 45, dans laquelle lesdites applications biologiques et médicales sont choisies dans le groupe constitué par le marquage de cellules, la séparation de cellules, la libération contrôlée, le diagnostic, l'immobilisation d'enzymes, la purification de protéines, la délivrance de médicaments, des agents de contraste pour une IRM et des applications de formation d'images sono et la chélation d'ions métalliques lourds.
